# EUROPEAN PATENT SPECIFICATION

(11) **EP 2 332 428 B1**
(45) Date of publication and mention of the grant of the patent: **20.08.2014**
(21) Application number: 09015087.1
(22) Date of filing: 04.12.2009
(51) Int. Cl.: A23L 1/305, A61K 38/01

(54) **Nutritional Formulation comprising a cow's milk peptide-containing hydrolysate and/or peptides derived thereof for tolerance induction**
Nahrungsformulierung mit kuhmilchpeptidhaltigem Hydrolysat und/oder Peptiden daraus für Toleranzinduktion
Formule nutritionnelle comportant un hydrolysat contenant un peptide de lait de vache et/ou des peptides dérivés pour une induction à la tolérance

(43) Date of publication of application: 15.06.2011
(73) Proprietor: MJN U.S. Holdings LLC, Glenview, IL 60026 (US)
(72) Inventor: Valenta, Rudolph, 1090 Wien (AT); Hochwallner, Heidrun, 3243 St. Leonard/Forst (AT); Focke-Tejkl, Margarete, 1140 Wien (AT); Swoboda, Ines, 1020 Wien (AT); Van Tol, Eric, 6824 MZ Arnhem (NL); Herz, Udo, 35274 Kirchhain (DE); Schulmeister, Ulrike, 3914 Waldhausen (AT)
(74) Representative: Vossius & Partner

(56) References cited:
- WO-A1-2008/056983
- DE-A1-102004 040 452
- US-A- 6 077 558
- RUITER B ET AL.: "Characterization of T cell epitopes in alpha s1-casein in cow's milk allergic, atopic and non-atopic children" CLINICAL AND EXPERIMENTAL ALLERGY, vol. 36, 2006, pages 303-310, XP002582387 DOI: 10.1111/j.1365-2222.2006.02436.x
- KAZUKI HIRAHARA ET AL.: "Profound immunological tolerance in the antibody response against bovine alpha s1-casein induced by intradermal administration of a dominant T cell determinant" CLINICAL IMMUNOLOGY AND IMMUNOPATHOLOGY, vol. 76, no. 1, 1995, pages 12-18, XP002582388 DOI: 10.1006/clin.1995.1082
- SCHULMEISTER ULRIKE ET AL: "Cloning, Expression, and Mapping of Allergenic Determinants of alpha S1-Casein, a Major Cow's Milk Allergen" JOURNAL OF IMMUNOLOGY, vol. 182, no. 11, 1 June 2009 (2009-06-01), pages 7019-7029, XP002563141 AMERICAN ASSOCIATION OF IMMUNOLOGISTS, US ISSN: 0022-1767 DOI: 10.4049/JIMMUNOL.0712366
- ANDREA VON BERG ET AL.: "The effect of hydrolyzed cow's milk formula for allergy prevention in the first year of life: the german infant nutritional intervention study, a randomized double-blind trial" JOURNAL OF ALLERGY AND CLINICAL IMMUNOLOGY, vol. 11, 2003, pages 533-540, XP002603102
- Lake, Alan M.: "Food-Induced Eosinophilic Proctocolitis"[Online] vol. 30, no. 1, January 2000 (2000-01), page 5 PP, XP002603103 Journal of Pediatric Gastroenterology & Nutrition Retrieved from the Internet: URL:http://journals.lww.com/jpgn/fulltext/ 2000/01001/food_induced_eosinophilic_proct ocolitis.9.aspx> [retrieved on 2010-10-07]
- JANET L SMITH: "Increased Ureagenesis and Impaired Nitrogen Use during Infusion of a Synthetic Amino Acid Formula A Controlled Trial" NEW ENGLAND JOURNAL OF MEDICINE,, vol. 306, no. 17, 1 January 1982 (1982-01-01), pages 1013-1018, XP008127498 THE, MASSACHUSETTS MEDICAL SOCIETY, WALTHAM, MA, US ISSN: 0028-4793

## Description

The present invention relates in one embodiment to a nutritional formulation or supplement comprising an extensively hydrolyzed cow's milk peptide-containing hydrolysate and/or a peptide-containing fraction of the extensively hydrolyzed cow's milk peptide-containing hydrolysate and/or one or more T cell epitone-containing peptides isolated from the extensively hydrolyzed cow's milk peptide-containing hydrolysate for use in the induction of tolerance to cow's milk, a protein contained in cow's milk or an allergen contained in cow's milk in a human subject, wherein said peptides contained in the hydrolysate or fraction of hydrolysate comprise T cell epitope-containing peptides or wherein said one or more peptides are T cell epitope-containing peptides, wherein said T cell epitope-containing peptides are capable of driving the immune reaction upon intake of the nutritional formulation towards tolerance, and wherein the extensively hydrolyzed cow's milk peptide-containing hydrolysate comprises less than 1 % of peptides having a size of greater than 1.5kD.

Cow's milk allergy is one of the most common food allergies in young children, with approximately 2% to 2.5% of all infants experiencing allergic reactions to milk. The majority of children out-grow their allergy to cow's milk before the age of 3, but 15% of these infants with retain their sensitivity to cow's milk into the second decade of life. Therefore, subjects having a cow's milk allergy are present in all age groups. Allergic diseases like milk allergy are immunological disorders which originate from the activation of a subset of T cells secreting allergic, inflammatory factors including IL-4, IL-5 and/or IL-13 (Schmidt-Weber et al., Allergy 2002, Vol 57, pp 762-768). This subset of T cells controls the isotype switching of antigen-specific B cells to IgE and therefore plays a key role in the initiation of allergic symptoms, as well as in tolerance induction (Kondo et al., Pediatr Allergy lmmunol. 2008, Vol. 19, pp 592-598). Hence, the regulation of allergen-specific T cells is a promising strategy to control allergic diseases.

In order to avoid allergic reactions upon exposure to cow's milk in a cow's milk allergic subject, and in particular in infants having a cow's milk allergy, mostly milk substitute formulas are presently used which replace nutrition with cow's milk. These formulas additionally provide the subject with a complete source of nutrition. Milk substitutes include free amino acids (such as Nutramigen^{™} AA, Neocate), soy based formulas (such as Pregomin), or hypoallergenic formulas based on partially or extensively hydrolyzed protein (such as Nutramigen^{™}, Alimentum, and Pregestemil). United States Patent No. 6.077.558 describes an emulsifying system for elemental diet compositions comprising a protein source that may be an extensively hydrolyzed protein, free amino acids, short-chain-peptides, or a mixture thereof. WO 2008/056983 refers to probiotic infants and children's food and dietary supplements that contribute to general resistance to pathogens and the incidence of respiratory diseases in children. The supplements may include a casein hydrolysate, such as, for instance, a non-allergenic low-fat extensively hydrolyzed casein hydrolysate. Ruiter, B. et al., Characterization of T cell epitopes in αs1-casein in cow's milk allergic, atonic and non-atopic children, Clinical and Experimental Allergy, 36, 303-310 (2006) relates to the identification of T cell epitopes that may be useful targets in developing peptide immunotherapy. Lake, Alan M., Food-Induced Eosinophilic Proctocolitis, Journal of Pediatric Gastroenterology Nutrition, Volume 30, Issue 1, pp. S58-S60, January (2000) describes that an extensively hydrolyzed casein-based formulation_can be given to infants with eosinophilic procolitis in order to remove offending protein from the diet. If allergic subjects do not respond to protein hydrolysate formulas, non-milk derived amino acid-based formulas are suitable for the treatment of both mild-moderate and severe milk allergy. Soy based formulas have a risk of allergic sensitivity, as some subjects who are allergic to milk may also be allergic to soy. Partial hydrolysate formulas are characterized by a larger proportion of long amino acid chains (peptides) compared to extensive hydrolysates and are considered more palatable. They are usually intended for prophylactic use and are generally not considered suitable for treatment of milk allergy/intolerance. Extensively hydrolysed proteins, on the other hand, comprise predominantly free amino acids and short peptides. Casein and whey are the most commonly used sources of protein for hydrolysates because of their high nutritional quality and their amino acid composition.

Hence, most of today's cow's milk substitute formulas on the market are based on cow's milk that has been hydrolyzed to various degrees and/or on amino acid formulations. These cow's milk formulas are used to replace cow milk and thereby reduce allergic reactions in cow's milk allergic subjects. Moreover, cow's milk formulas can potentially prevent the development of cow's milk allergy in a subject being at risk of developing a milk allergy. However, even extensively hydrolyzed products have occasionally been observed to elicit allergic reactions in sensitized infants (Rosendal et al. Journal of Dairy Science 2000, Vol. 83, No. 10, pp 2200-2210).

The current treatment for milk allergies is therefore the total avoidance of cow's milk and food. Consequently, a substance which could drive the immune reaction upon intake of the substance towards tolerance to cow's milk or food comprising cow's milk would drastically increase the quality of life of a subject having cow's milk allergy or having the risk of developing a cow's milk allergy. This need is addressed by the present invention.

The present invention is defined by the claims. Accordingly, the present invention relates In one embodiment to a nutritional formulation or supplement comprising an extensively hydrolyzed cow's milk peptide-containing hydrolysate and/or a peptide-containing fraction of the extensively hydrolyzed cow's milk pepotide-containing hydrolysate and/or one or more T cell epitope-containing peptides isolated from the extensively hydrolyzed cow's milk peptide-containing hydrolysate for use in the induction of tolerance to cow's milk, a protein contained in cow's milk or an allergen contained in cow's milk in a human subject, wherein said peptides contained in the hydrolysate or fraction of hydrolysate comprise T cell epitope-containing peptides or wherein said one or more peptides are T cell epitope-containing peptides, wherein said T cell epitope-containing peptides are capable of driving the immune reaction upon intake of the nutritional formulation towards tolerance, and wherein the extensively hydrolyzed cow's milk peptide-containing hydrolysate comprises less than 1% of peptides having a size of greater than 1.5kD.

The term "nutritional formulation" as used herein describes a solid or liquid formulation which can therefore be eaten or drunk by a human subject for nutrition. The nutritional formulation of the invention preferably has a nutritional value of at least 1, more preferred at least 10 and even more preferred 50 kcal(kilo calorie)/100ml for liquid formulations and preferably at least 1, more preferred at least 10, even more preferred at least 50, such as at least 100, and most preferred at least 300 kcal/100g for dry food formulations. In a preferred embodiment of the invention the nutritional formulation of the invention has a nutritional value of at least 50-200 kcal/100ml for liquid formulations and at least 300-600 kcal/100g for dry food formulations. A nutritional formulations is distinguished from a vaccine. In contrast to a vaccine, a nutritional formulation does not comprise any of adjuvants (unless as contaminations), activated or inactivated viral compounds (unless as contaminations), activated or inactivated bacterial compounds (unless as contaminations), and pathogenic compounds (unless as contaminations).

The term "supplement" as used herein relates to a nutritional supplement which is a concentrated source of nutrient or alternatively other substances with a nutritional or physiological effect whose purpose is to supplement the normal diet.

In addition to the above recited ingredients further ingredients may be selected from lipids, minerals, carbohydrates, amino acids, amino acid chelates, anabolic nutrients, vitamins, antioxidants, probiotic bacterial strain and lipotropic agents in order to provide an optimal sustained energy and anabolic nutritional formulation. The nutritional formulation may be a nutritional supplement or may provide complete nutrition. Preferably the nutritional formulation is in the form of a dry food concentrate. The nutritional formulation of the invention provides a human subject with increasing preference with at least 5%, at least 10%, at least 25%, at least 50%, at least 75% or at least 90% of the daily calorie requirement of a human subject. The person skilled in the art is well aware that the daily calorie requirement is dependent on the gender, height and age of a human subject. For example, a 30 year old male of 80kg body weight and 180cm height has a daily calorie requirement of around 2900 cal (calories) to maintain his body weight whereas a 30 year old female of 55kg body weight and 165cm height has a daily calorie requirement of around 2100 cal to maintain her body weight. In a preferred embodiment, the nutritional formulation of the present invention is an infant or a nutritional product for infants or juvenile.

The term "peptide" as used herein describes linear molecular chains of amino acids, including single chain molecules or their fragments. A peptide in accordance with the invention contains with increasing preference about 2 to 100 amino acids, about 5 to 50 amino acids, or about 5 to 40 amino acids. Peptides may further form oligomers consisting of at least two identical or different molecules. The corresponding higher order structures of such multimers are, correspondingly, termed homo- or heterodimers, homo- or heterotrimers etc. Furthermore, peptidomimetics of such peptides where amino acid(s) and/or peptide bond(s) have been replaced by functional analogs are also encompassed by the term "peptide". Such functional analogues include all known amino acids other than the 20 gene-encoded amino acids, such as selenocysteine. The term "peptide" also refers to naturally modified peptides where the modification is effected e.g. by glycosylation, acetylation, phosphorylation and similar modifications which are well known in the art. A peptide has to be distinguished from a protein in the present invention. A protein in accordance with the present invention describes an organic compound made of amino acids arranged in a linear chain and folded into a globular form. Furthermore, a protein in accordance with the present invention describes an amino acids of more than 100 amino acids. Peptides may, e.g., be produced recombinantly, (semi-)synthetically, or obtained from natural sources such as after hydrolysation of proteins, all according to methods known in the art.

The term "cow's milk peptide-containing hydrolysate" as used herein defines a formula which comprises peptides derived from hydrolyzed cow's milk proteins (e.g. bovine casein or bovine whey). In this regard, a hydrolyzed protein is a protein that has been broken down into peptides and/or component amino acids. While there are many means of achieving protein hydrolysis, two of the most common means are prolonged boiling in a strong acid or strong base or using an enzyme such as the pancreatic protease enzyme to stimulate the naturally-occurring hydrolytic process. Hydrolysis of proteins derived from milk is preferably achieved using an enzyme or a mixture of enzyme. A cow milk hydrolysate can comprise peptides derived from milk, wherein the proteins of said milk have been hydrolyzed to various degrees. Accordingly, one can distinguish between a partially hydrolyzed cow's milk peptide-containing hydrolysate and an extensively hydrolyzed cow's milk peptide-containing hydrolysate. In this regard, a partially hydrolyzed cow's milk peptide-containing hydrolysate comprises more than 20% of intact cow's milk protein whereas an extensively hydrolyzed cow's milk peptide-containing hydrolysate comprises less than 1% of peptides having a size of greater than 1.5kD. Furthermore, an extensively hydrolyzed cow's milk peptide-containing hydrolysate is preferably hypoallergenic.

The term "peptide derived from cow's milk" as used herein defines a peptide which has an amino acid sequence which is a partial amino acid sequence of a cow's milk protein. Such peptides may be obtained as outlined above by hydrolysis or may be synthesized in vitro by methods known to the skilled person and described in the examples of the invention.

The term "T cell epitope-containing peptide" in accordance with the invention describes a peptide which comprises an epitope which is capable to bind to a surface receptor which is present on a T-cell. It is preferred that the epitope is capable to bind to a T cell receptor (TCR).

The term "peptide-containing fraction of the hydrolysate" refers to a mixture of peptides comprising at least 2, preferably at least 5, more preferably at least 10 and most preferably at least 20 which have been isolated from the hydrolysate of the invention by filtration techniques which are known to the skilled person. Furthermore, techniques for the isolation of peptides from the hydrolysate of the invention are described herein below.

The term "tolerance" according to the invention refers to an immunological tolerance. Immunological tolerance is defined herein as the development of specific non-reactivity or partial non-reactivity of lymphoid tissues to a particular antigen or group of antigens. This particular antigen or group of antigens is capable of inducing an immune reaction in an atopic human subject upon intake of the particular antigen or group of antigens. Accordingly, tolerance to the particular antigen or group of antigens in accordance with the present invention is induced upon intake of one or more T cell epitope-containing peptides of the invention. Without wishing to be bound by theory, tolerance may be induced, for example, by mechanism of anergy and deletion of specific allergen-reactive T cells. Alternatively, tolerance may, for example, be induced by cellular factor (i.e. cytokines) which could drive the T cell development into the direction of the development of a tolerogenic or suppressive T cell phenotype (regulatory T cells).

In this regard, the term "T cell epitope-containing peptides capable to drive the immune reaction upon intake of the nutritional formulation towards tolerance" specifies peptides which contribute to the development of specific non-reactivity of lymphoid tissues to a particular antigen or group of antigens, wherein this activity of the peptide is achieved by binding to a T cell surface receptor. Accordingly, the T cell epitope-containing peptides are present in an amount in the nutritional formation which allows for driving the immune reactions towards tolerance. It is preferred that the immune reaction is reduced by the induction of tolerance by at least 20%, such as at least 50%, such as by at least 75%, preferably by at least 90%, more preferably by at least 95%, and most preferably by 100% as compared to the immune reaction upon contact with the antigen without prior tolerance induction. Methods for measuring tolerance are known in the art and include for example the methods described in the examples of the invention. Accordingly, tolerance may be determined for example by measuring the amount of proinflammatory factors released from T cells (e.g. interleukins or interferones) or the proliferation of T cells. A peptide which is a T cell epitope-containing peptide capable of driving the immune reaction upon intake of the nutritional formulation towards tolerance can be indentified by methods known to the skilled person in the art which are, for example, described in the examples of the invention herein below. Accordingly, a peptide which can inhibit the proliferation of T cells, and/or downregulates the release of pro-inflammatory cytokines and/or cytokines driving T helper cell 2 (TH2) differentiation released in the supernatant of PBMCs is a T cell epitope-containing peptide capable of driving the immune reaction upon intake of the nutritional formulation towards tolerance.

In accordance with the present invention, it has surprisingly been found that T cell epitope-containing peptides contained in cow's milk hydrolysate and T cell epitope-containing peptides which are derived from a protein present in cow's milk can be used in the induction of tolerance As is evident from the examples below, the inventors have found that peptides contained in milk hydrolysates and peptides derived from a protein present in cow's milk can drastically decrease proliferation of T cells when there is a subsequent exposure to milk allergens, presumably via blocking T cell receptors and MHCII binding. Specific peptides contained in milk hydrolysates or synthetic peptides corresponding to a partial sequence of a cow's milk peptide can even block proliferation of T cells when there is a subsequent exposure to milk allergens. Whereas T-cell epitopes in cow's milk proteins have been described in the art (e.g. Kondo et al., Pediatr Allergy Immunol. 2008, Vol. 19, pp 592-598; Elsayed et al., Mol Immunol. 2004, Vol 41(12), pp1225-34 ; Ruiter et al., Clin Exp Allergy. 2006, Vol 36(3), pp 303-10; Ruiter et al., lnt Arch Allergy lmmunol. 2007; Vol 143(2), pp119-26; or Nakajima-Adachi et al., J Allergy Clin Immunol. 1998; Vol 101 (5), pp 660-71), it has not been described previously that cow's milk derived peptides can induce tolerance. Further, in accordance with the present invention it has also been found that T cell epitope-containing peptides contained in cow's milk hydrolysate and T cell epitope-containing peptides which are derived from a protein present in cow's milk reduce levels of cytokines driving a T-helper cell 2 (TH2) differentiation and levels of pro-inflammatory cytokines as compared to levels of said cytokines induced by milk proteins. As known in the field on immunology, pro-inflammatory cytokines have detrimental effects on the intestinal barrier integrity and therefore are involved in the development of allergic as well as inflammatory disease. Amongst pro-inflammatory cytokines in particular IL-4 and IFN-gamma are known to destruct the coherence between the epithelial cells lining the gut surface thus compromising the intestinal barrier integrity. As a results of this the intestine becomes more permeable with increased exposure of allergens and dietary/microbial antigens to the immune cells in the gut wall.

Thus, without wishing to be bound by theory, tolerance is believed to be induced directly by cow's milk derived peptides. Previously cow's milk hydrolysates and peptides have only been used in order to replace cow's milk. By the replacement of cow's milk an allergic reaction or the development of an allergic reaction has been avoided. The findings provided herein show that that peptides which are T cell epitope-containing peptides contained in cow's milk hydrolysate and T cell epitope-containing peptides which are derived from a protein present in cow's milk can moreover and unexpectedly be used to induce tolerance in a human subject.

In accordance with the invention the tolerance is induced to cow's milk, a protein contained in cow's milk or an allergen contained in cow's milk.

Cow's milk, a protein contained in cow's milk or an allergen contained in cow's milk are, for example, comprised in any food comprising cow's milk ingredients. Non-limiting examples are milk, curd, cream, butter, yoghourt and food containing any of these.

Then term "allergen" as used herein describes an antigen capable of stimulating a hypersensitivity reaction in an atopic (allergic) human subject. Furthermore, an allergen is in general a substance that is foreign to the body and can cause an allergic reaction only in atopic human subjects.

Also described herein is a nutritional formulation or supplement comprising a cow's milk peptide-containing hydrolysate and/or peptide-containing fraction of the hydrolysate and/or one or more peptides derived from a protein present in cow's milk for use in treating or preventing inflammatory bowel disease, wherein said peptides contained in the hydrolysate or fraction of hydrolysate comprise T cell epitope-containing peptides or wherein said one or more peptides are T cell epitope-containing peptides, and wherein said T cell epitope-containing peptides are capable of downregulating pro-inflammatory cytokines upon intake of the nutritional formulation.

The term "inflammatory bowel disease" as used herein defines a group of inflammatory conditions of the gastrointestinal tract. The major types of inflammatory bowel disease are Crohn's disease and ulcerative colitis. The main difference between Crohn's disease and ulcerative colitis is the location and nature of the inflammatory changes. Crohn's disease can affect any part of the gastrointestinal tract, from mouth to anus (skip lesions), although a majority of the cases start in the terminal ileum. Ulcerative colitis, in contrast, is restricted to the colon and the rectum. In a preferred embodiment the inflammatory bowel disease is present in a human subject having a milk allergy or having the risk of developing a milk allergy. In a further preferred embodiment the inflammatory bowel disease is selected from one or more of Crohn's disease, ulcerative colitis, gastro esophageal reflux disease (GERD), gastroenteritis, colitis and/or esophagitis. GERD is a disease produced by the abnormal reflux in the esophagus. In particular, gastro esophageal reflux disease (GERD), gastroenteritis, colitis and/or esophagitis are common in human subjects having a cow's milk allergy.

As used herein, the term "pro-inflammatory cytokines" are cytokines which are released in a human subject by cells of the immune system, preferably by antigen presenting cells or T cells and most preferably by T cells and mediate and/or enhance an inflammatory disease. Non-limiting examples of inflammatory cytokines are IL-12, IL-17, IL-5, IL-4, IFN-γ, IL-8, TNF-α, IL-6 or IL-1. Methods for measuring the level of cytokine released by cells of the immune system are well know to the skilled person and include the methods described in the examples. Accordingly, the methods include, for example, measuring cytokine levels in culture supernatants.

The term "T cell epitope-containing peptides capable of downregulating pro-inflammatory cytokines upon intake of the nutritional formulation" refers to T cell epitope-containing peptides in the nutritional formulation which can downregulate the levels of pro-inflammatory cytokines which are released form immune cells, preferably T cells or APCs, and more preferably T cells. Furthermore, theses T cell epitope-containing peptides capable are administered to a human subject in amount which is sufficient to downregulate pro-inflammatory cytokines upon intake of the nutritional formulation. It is preferred that the level of pro-inflammatory cytokines are downregulated by at least 20%, such as at least 50%, such as by at least 75%, preferably by at least 90%, more preferably by at least 95%, and most preferably by 100% as compared to the level of pro-inflammatory cytokines in an atopic immune reaction upon contact with the antigen. A peptide which is a T cell epitope-containing peptides capable of downregulating pro-inflammatory cytokines upon intake of the nutritional formulation can be indentified by methods known to the skilled person in the art which are, for example, described in the examples of the invention herein below. Accordingly, measuring the level of pro-inflammatory cytokines released In the culture supernatant of PBMCs upon exposure to a peptide identifies a T cell epitope-containing peptide capable of downregulating pro-inflammatory cytokines upon intake of the nutritional formulation.

As described herein it has been surprisingly found that T cell epitope-containing peptides of the invention derived from cow's milk are capable of downregulating inflammatory cytokines that are released upon exposure to or stimulation by allergens or dietary/microbial antigens. Therefore, the nutritional formulation of the invention is suitable to treat inflammatory bowel disease.

In accordance with the invention the one or more peptides are T cell epitope-containing peptides isolated from the cow's milk peptide-containing hydrolysate.

As detailed above, the cow's milk peptide-containing hydrolysate comprises peptides derived from hydrolyzed cow's milk proteins. These peptides may be isolated from the milk peptide-containing hydrolysate by standard technique well known to the person skilled in the art. Accordingly, an analytical purification generally utilizes at least one of three properties to separate peptides. First, peptides may be purified according to their isoelectric points by running them through a pH graded gel or an ion exchange column. Second, peptides can be separated according to their size or molecular weight via size exclusion chromatography or by SDS-PAGE (sodium dodecyl sulfate-polyacrylamide gel electrophoresis) analysis. Third, another peptide purification methodology could involve membrane filtration (e.g. Ultra filtration) which would generate a mixture of peptides suitable for the proposed application. Peptides are often purified by using 2D-PAGE and are then analyzed by peptide mass fingerprinting to establish the peptide identity. The isolated and identified peptide obtained from the hydrolysate may then be recombinantly produced by applying standard methods known to the skilled person and either be used alone or in combination with a cow's milk peptide-containing hydrolysate in accordance with the invention.

A preferred embodiment of the invention relates to the nutritional formulation of the invention, wherein the one or more peptides have the amino acid sequence of SEQ ID NO: 3 and/or 4.

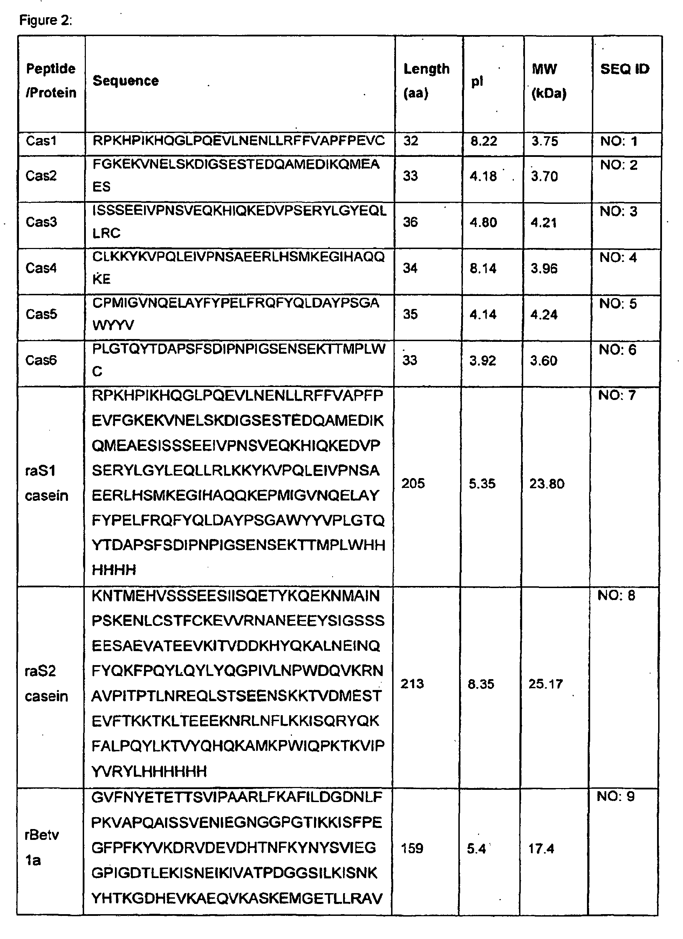

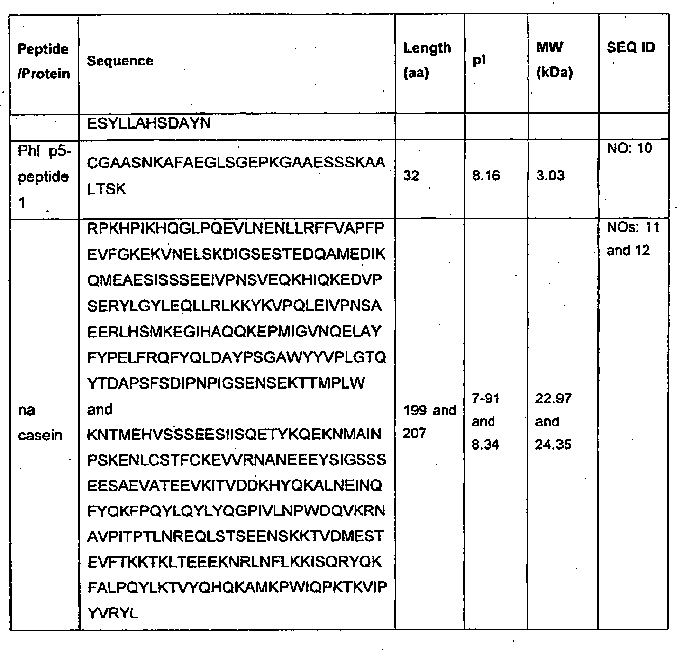

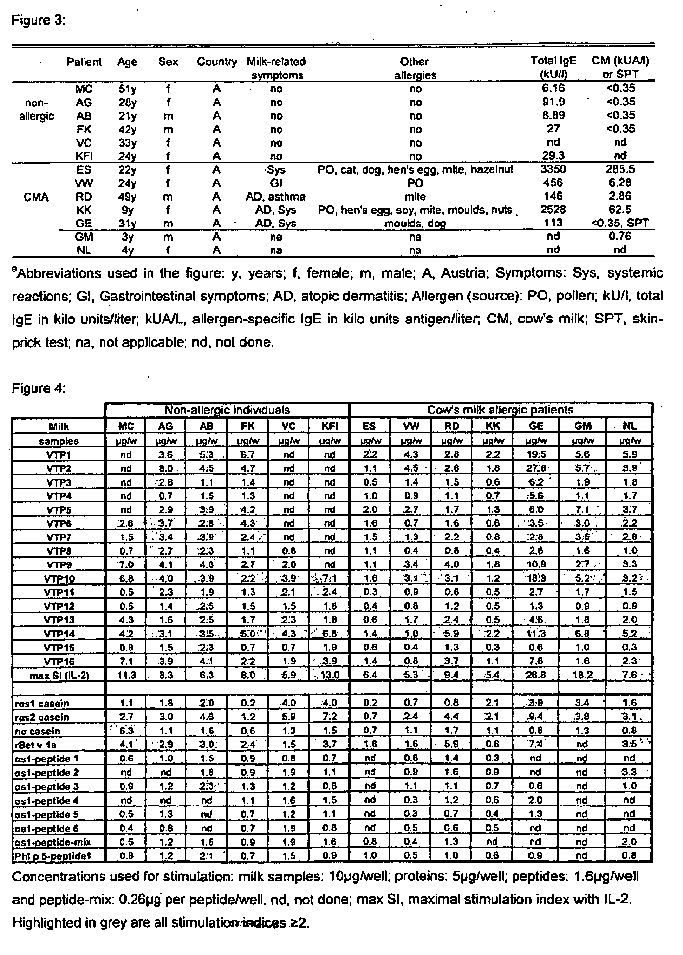

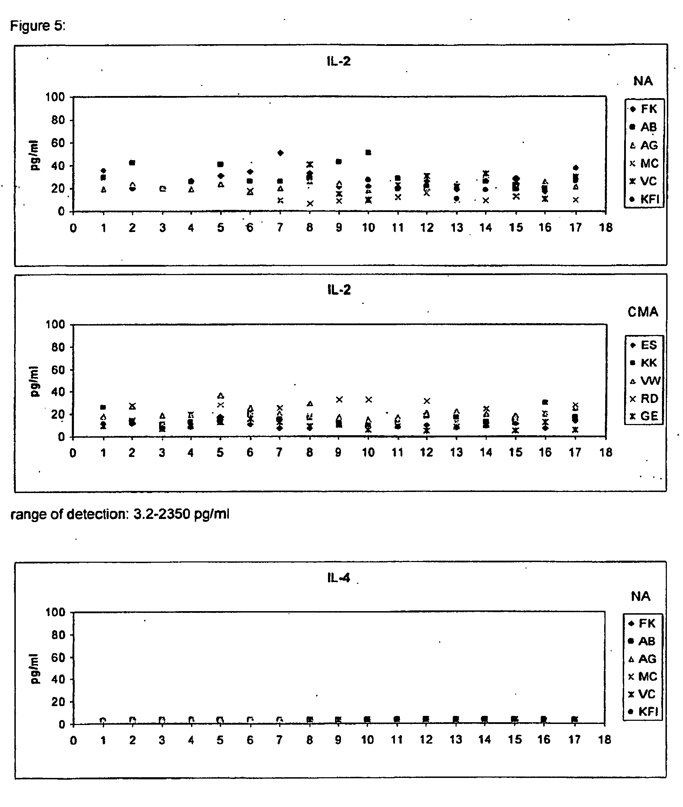

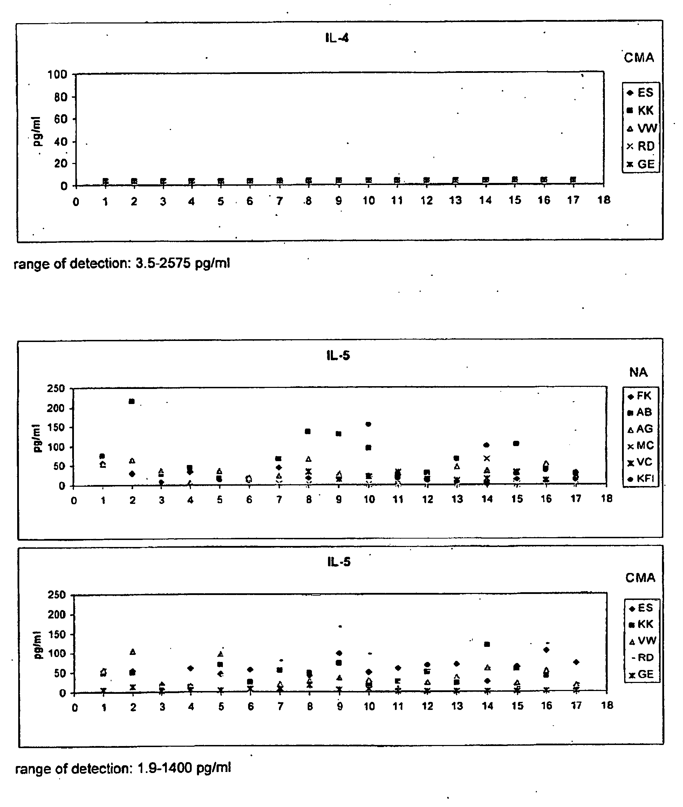

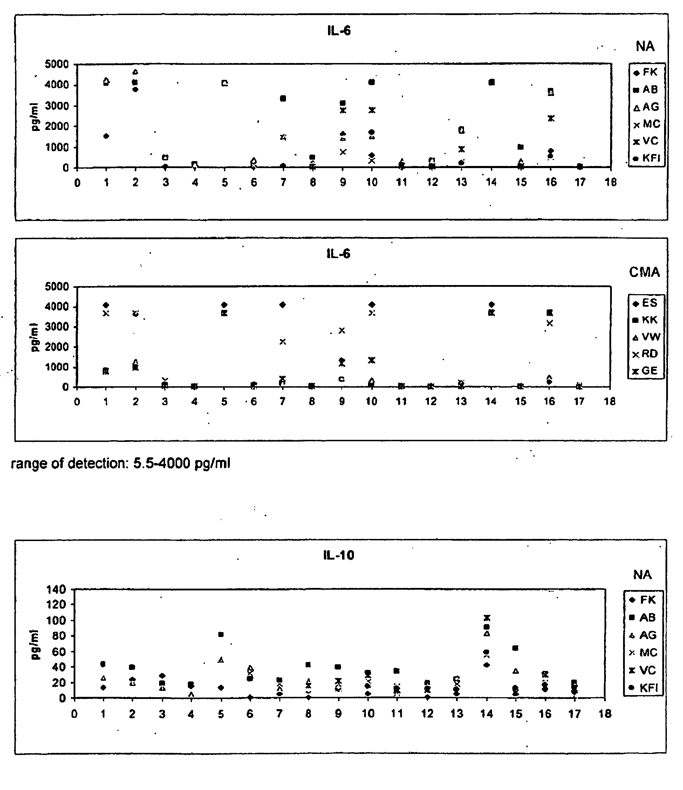

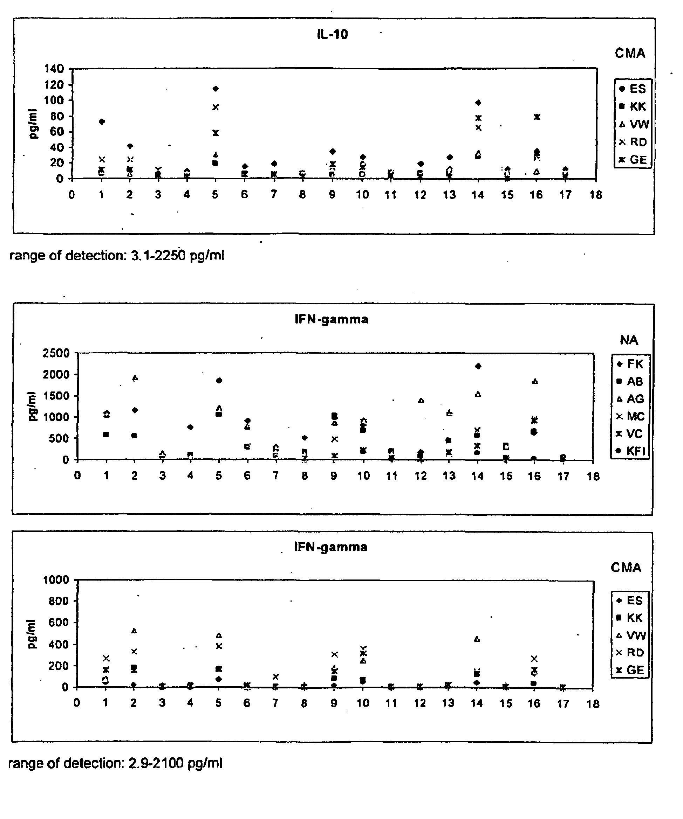

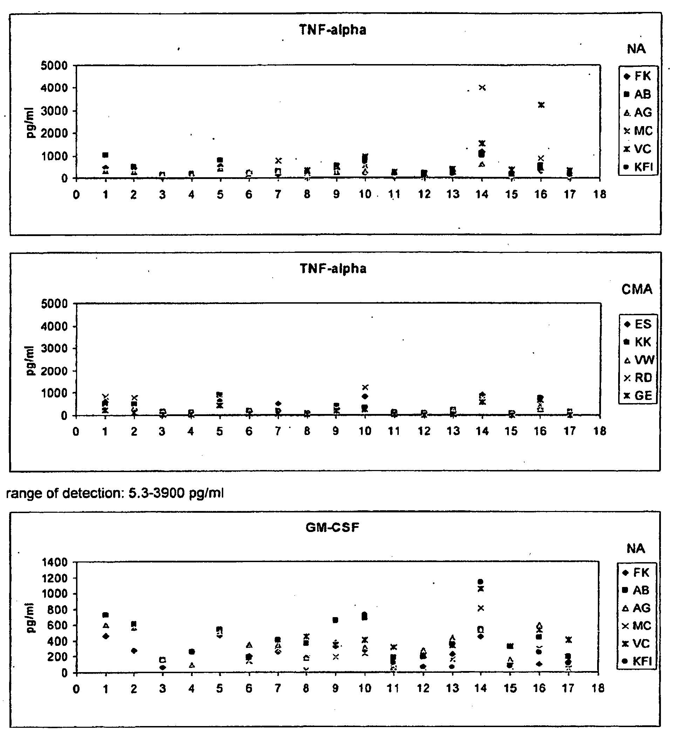

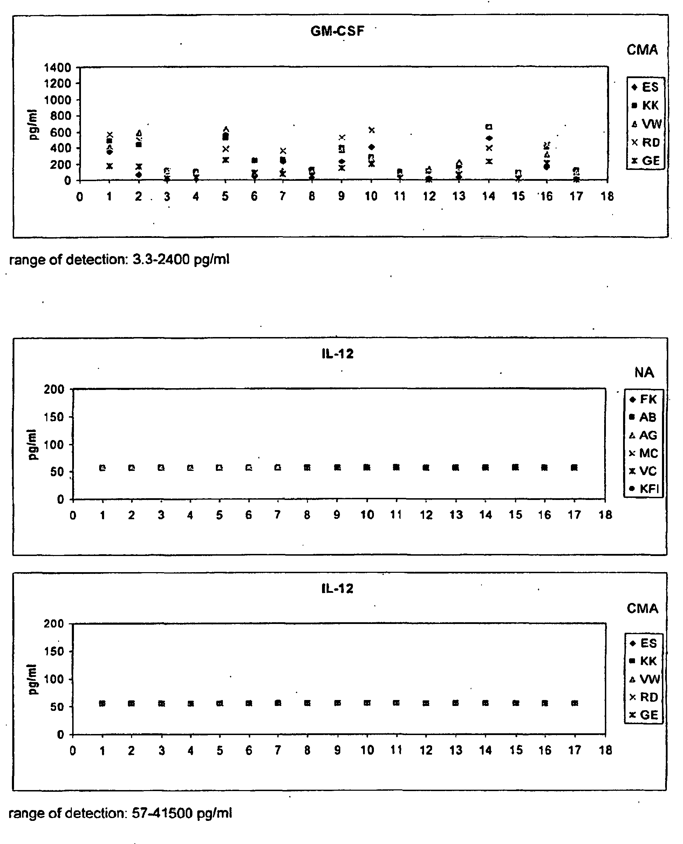

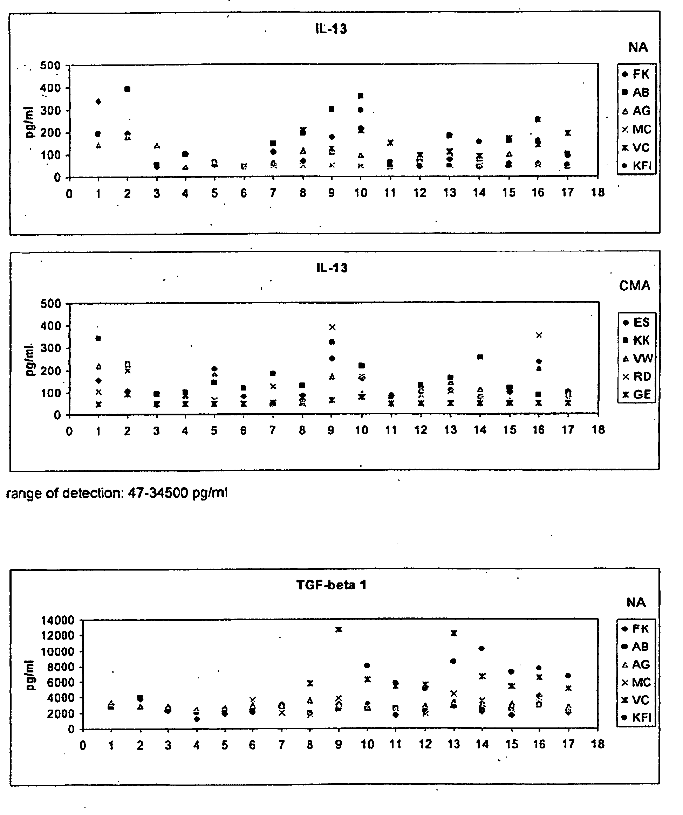

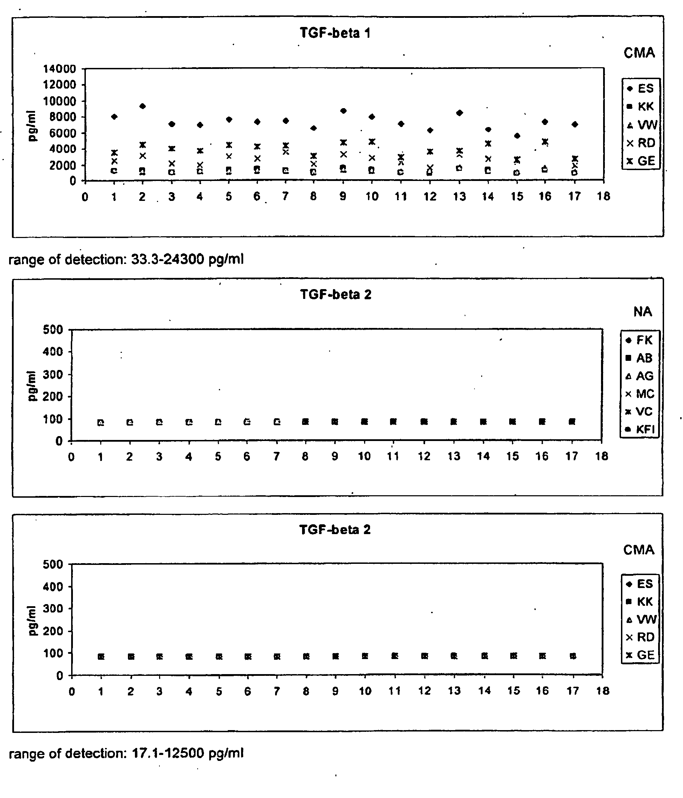

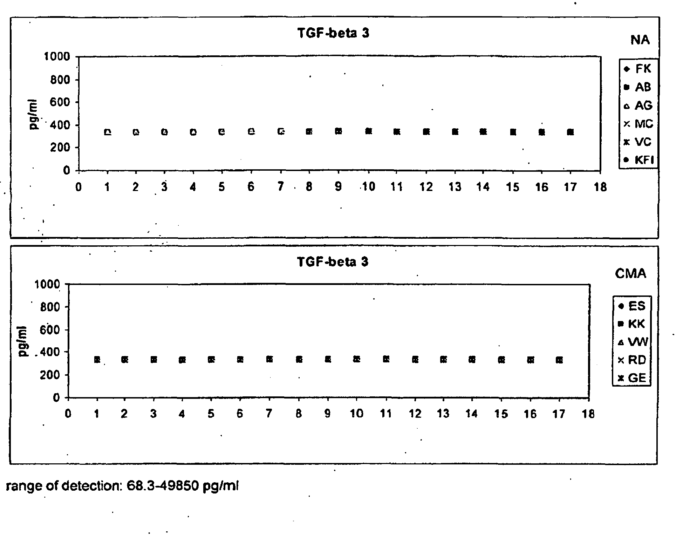

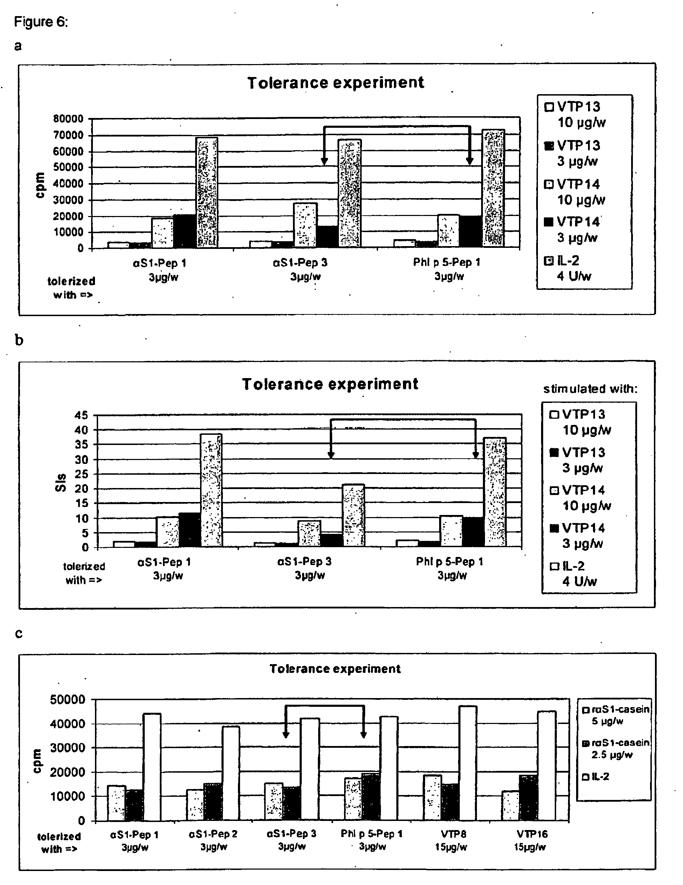

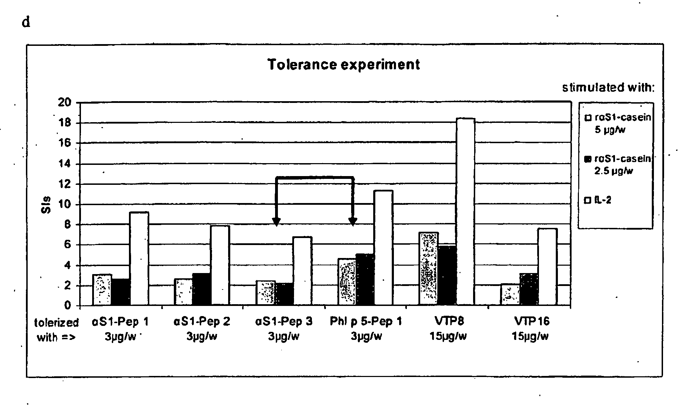

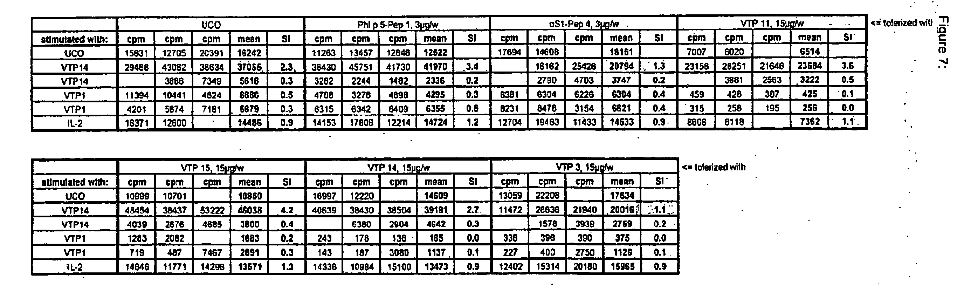

As shown in the examples below, the amino acid sequence of SEQ ID NO: 3 (Figure 2, Cas3, alphaS1-peptide 3) and SEQ ID NO: 4 (Figure 2, Cas4, alphaS1-peptide 4) suppress the proliferation of T-cells when re-stimulated with cow's milk protein and thereby induced tolerance to the cow's milk protein (cf. Figure 4).

In accordance with the invention the cow's milk peptide containing hydrolysate is an extensively hydrolyzed cow's milk peptide-containing hydrolysate.

A preferred embodiment of the invention relates to the nutritional formulation of the invention, wherein the extensively hydrolyzed cow's milk peptide-containing hydrolysate is an extensively hydrolyzed bovine casein hydrolysate.

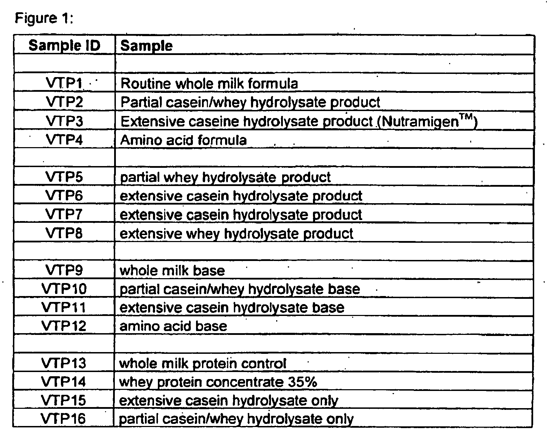

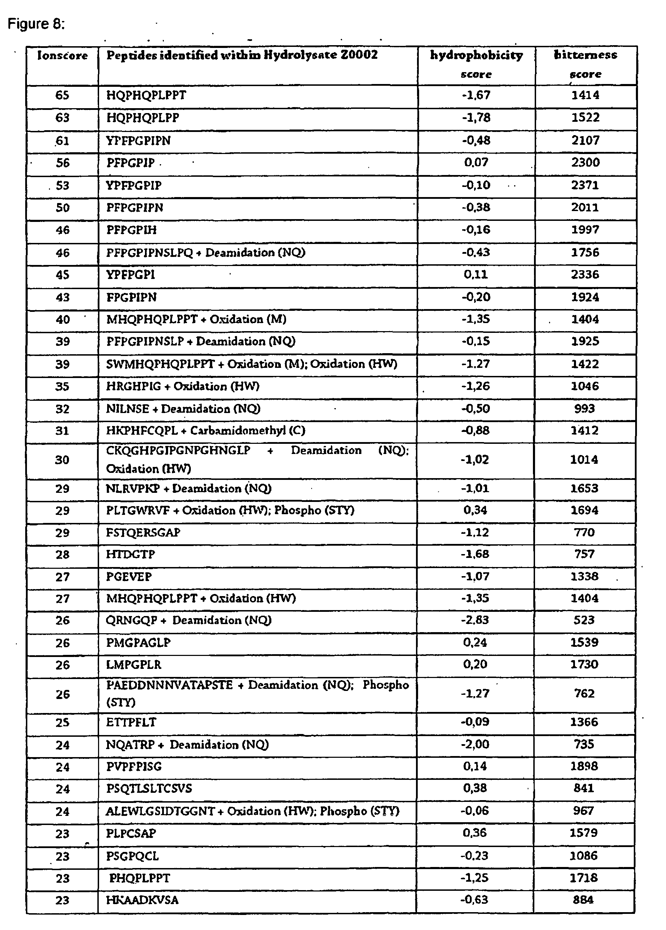

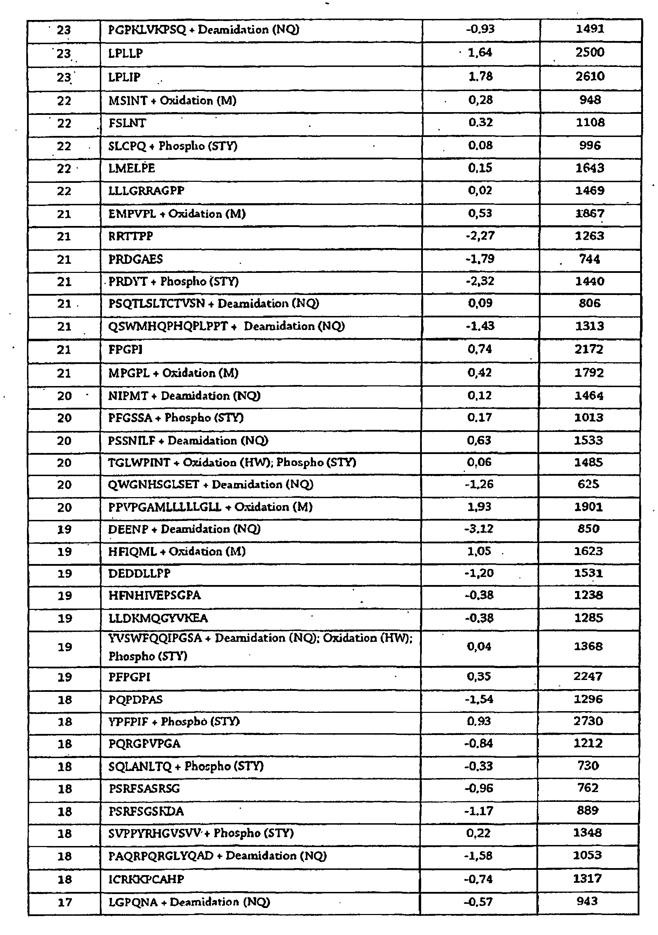

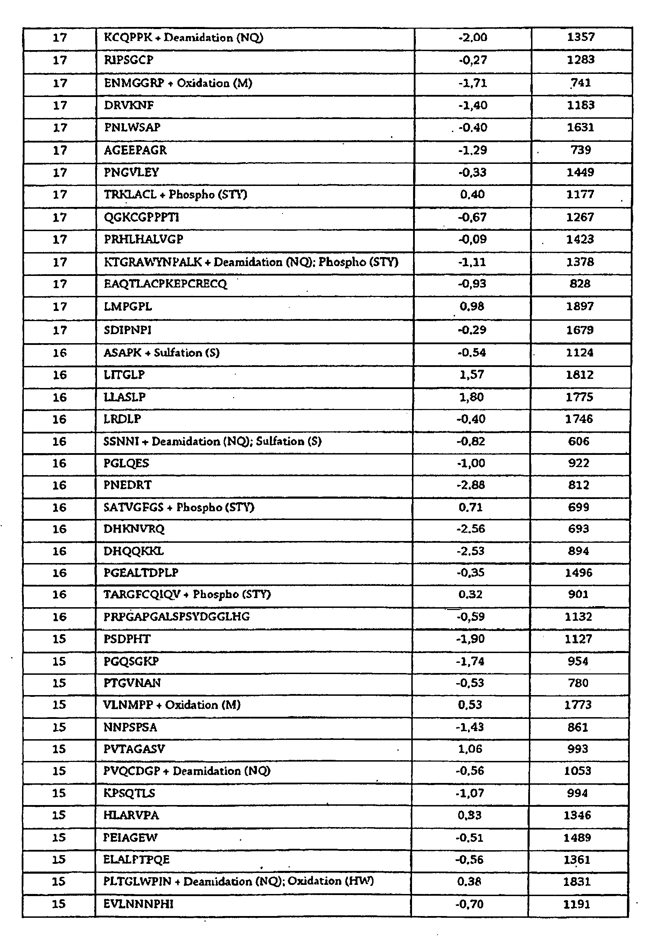

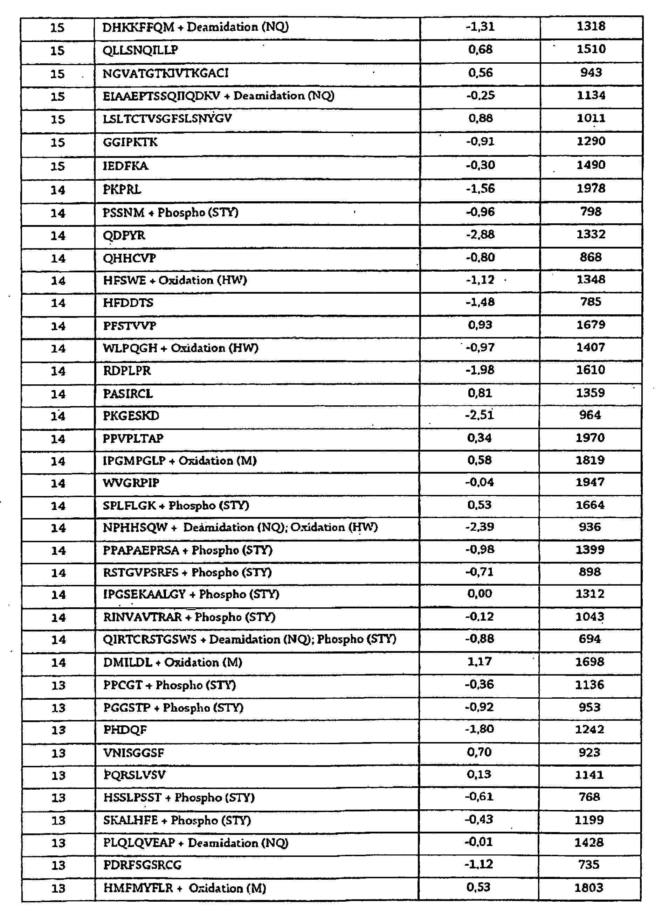

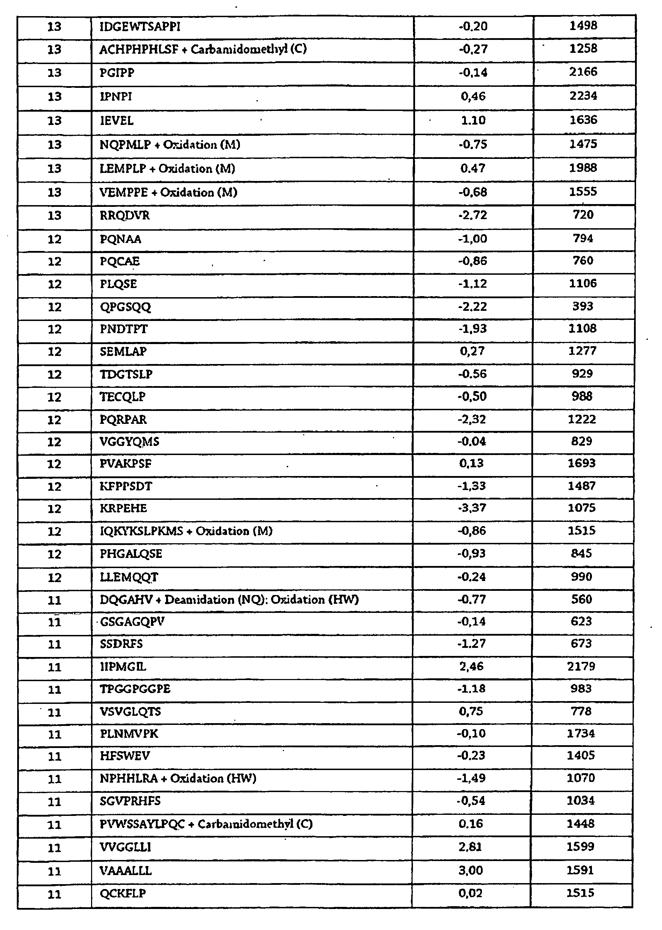

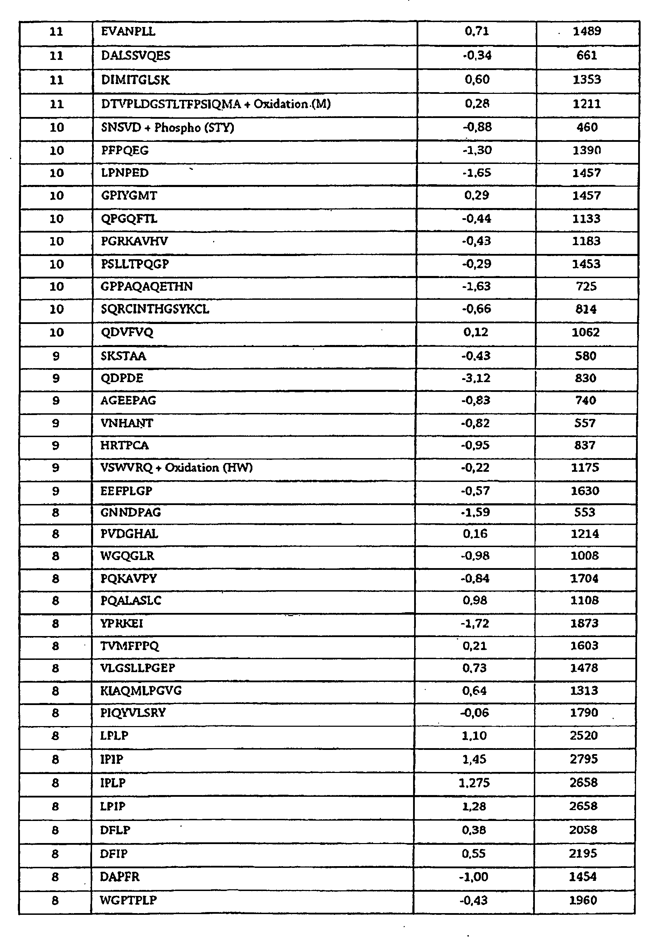

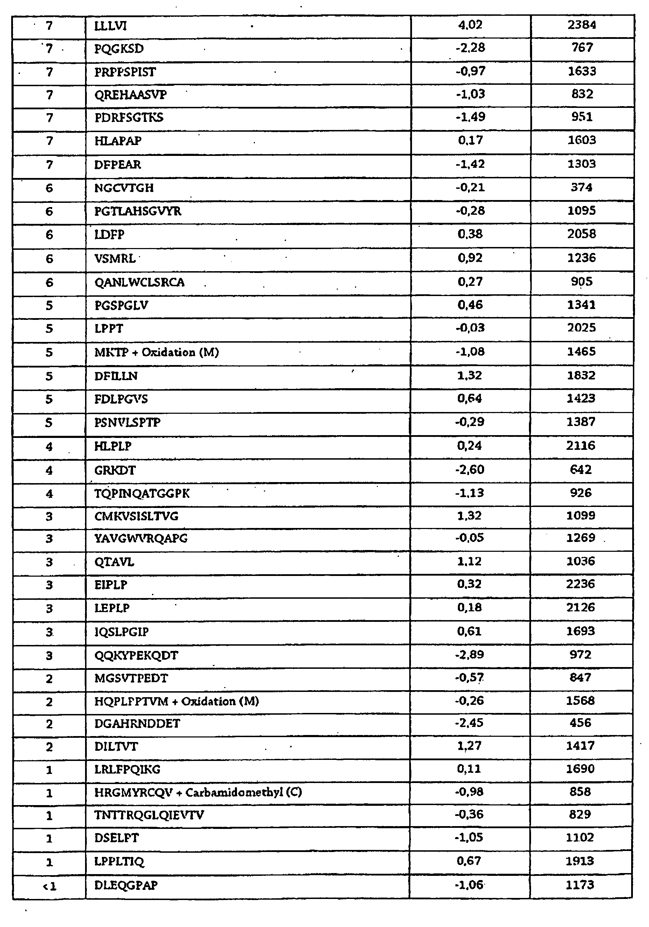

As is shown in the Examples of the invention below an extensively hydrolyzed cow's milk peptide-containing hydrolysate is able to suppress the proliferation of T-cells when these are re-stimulated with cow's milk protein. Tolerance to the cow's milk protein is thereby induced. Accordingly, VTP 3, VTP11 and VTP15 (cf. Figure 1) were most potent to induce tolerance and are, therefore, preferred examples of a nutritional formulation comprising an extensively hydrolyzed cow's milk peptide-containing hydrolysate in accordance with the invention, VTP3 is Nutramigen^{™}, VTP11 is Nutramigen^{™} base and VTP15 is the extensively hydrolyzed bovine casein that is used in Nutramigen^{™}. VTP3, VTP 11 and VTP 15 can be purchased via Mead Johnson Nutrition, 2400 West Lloyd Expressway Evansville, Indiana 47721-0001, USA. VTP3, VTP 11 and VTP 15 comprise extensively hydrolyzed bovine casein. A profile of the casein derived peptides comprised in VTP3, VTP 11 and VTP 15 is provided in Figure 8. VTP15 only consist of the peptides listed in Figure 8. Nutramigen^{™} (VTP3) comprises further to the peptides listed in Figure 8 the following ingredients: Glucose syrup, vegetable oil (palm olein oil, coconut oil, soybean oil, high oleic sunflower oil), modified corn starch, <2 %, calcium phosphate <1%, calcium citrate, potassium citrate, potassium chloride, L-Cystine, choline chloride, L-tyrosine, inositol, magnesium oxide, L-tryptophan, ascorbic acid, ferrous sulfate, taurine, L-carnitine, DL-alpha-tocopheryl acetate, zinc sulfate, nicotinamide, calcium pantothenate, cupric sulfate, retinyl palmitate, manganese sulfate, thiamine hydrochloride, riboflavin, pyridoxine hydrocholoride, sodium iodide, folic acid, phytomenadione, sodium molybdate, chromic chloride, cholecalciferol, sodium selenite, biotin, cyanocobalamin.

A further preferred embodiment of the invention relates to the nutritional formulation of the invention, wherein the human subject is a child or juvenile.

The term "child" or the term "juvenile" is used herein in accordance with the definitions provided in the art. Thus, the term "child" means a human subject between the stages of birth and the age of about 10 and the term "juvenile" means a human subject between the age of about 10 and puberty (before sexual maturity).

The invention relates in a further preferred embodiment to the nutritional formulation of the invention, wherein the human subject is an adult.

The term "adult" is used herein in accordance with the definitions provided in the art. Thus, this term means a human subject after puberty (after sexual maturity).

A further preferred embodiment of the invention relates to the nutritional formulation of the invention, wherein the human subject has a cow's milk allergy.

The term "cow's milk allergy" describes a food allergy, i.e. an immune adverse reaction to one or more of the proteins contained in cow's milk in a human subject. The principal symptoms are gastrointestinal, dermatological and respiratory symptoms. These can translate into skin rashes, hives, vomiting, diarrhea, constipation and distress. The clinical spectrum extends to diverse disorders: anaphylactic reactions, atopic dermatitis, wheeze, infantile colic, gastro esophageal reflux disease (GERD), esophagitis, colitis gastroenteritis, headache/migraine and constipation.

In another preferred embodiment of the nutritional formulation of the invention, the nutritional formulation additionally comprises one or more of carbohydrates, nucleic acids, lipids, minerals, anabolic nutrients, vitamins, antioxidants, probiotic bacterial strains and lipotropic agents.

These additional compounds of the nutritional formulation of the invention are preferably added in order to provide the nutritional value of the nutritional formulation described herein above. Also they may be preferably added in order provide complete nutrition, an optimal sustained energy and/or an anabolic nutritional formulation. Non-limiting examples of lipids include coconut oil, soy oil, and mono- and diglycerides. Exemplary carbohydrates are for example, glucose, edible lactose and hydrolyzed cornstarch. Non-limiting examples of minerals and vitamins are calcium, phosphorous, potassium, sodium, chloride, magnesium, manganese, iron, copper, zinc, selenium, iodine, and Vitamins A, E, D, C, and the B complex, respectively. Probiotic bacterial strains include, for example, lactic acid bacteria (LAB) and bifidobacteria. Examples of antioxidants include natural antioxidants such as ascorbic acid (AA, E300) and tocopherols (E306), as well as synthetic antioxidants such as propyl gallate (PG, E310), tertiary butylhydroquinone (TBHQ), butylated hydroxyanisole (BHA, E320) and butylated hydroxytoluene (BHT, E321). Non-limiting examples of nucleic acids are deoxyribonucleic acid (DNA) and ribonucleic acid (RNA)

A further preferred embodiment of the invention relates to the nutritional formulation of the invention, wherein the tolerance is induced transiently.

The term "transient" induction of tolerance in accordance with the invention relates to a immunological tolerance which is induced for a limited period of time upon intake of the nutritional formulation of the invention in a human subject which preferably is a human subject having a cow's milk allergy or having the risk of developing a cow's milk allergy. In this regard a transient period of time specifies that the human subject does not gain constant tolerance. Transient tolerance preferably relates to tolerance induced for at least half a day, such as for at least one day, such as for at least two days, preferably for at least one week, more preferably for at least two weeks, even more preferably for at least one month and most preferably for at least 3 months. In accordance with the invention transient tolerance is induced for less than 6 months.

The Figures show:
Figure 1:
   Overview of the milk test samples named VTP1-VTP16 which were obtained from by different suppliers and contained whole cow's milk protein formulas, partlially hydrolyzed, and extensively hydrolyzed casein and whey proteins as well as amino acids cow's milk formulas as well as amino acids formulas.
Figure 2:
   Synthetic peptides and proteins. The name of the peptides or protein, their amino acid sequence, length, pl and molecular weight in kDa are listed.
Figure 3:
   Demographic, clinical and serological characterization of individuals analyzed in the proliferation assays.
Figure 4
   Proliferation assays were performed with PBMCs to test the T-cell reactivity to the milk samples VTP1-VTP16. PBMCs from 6 non-allergic individuals and from 7 cow's milk allergic patients were stimulated with milk samples, with cow's milk allergens (rαS1-casein, rαS2-casein, nα-casein), with αS1-casein-derived peptides (αS1-peptide 1- αS1-peptide 6), with birch pollen allergen rBet v 1a and grass pollen allergen Phl p 5-peptide 1. Stimulation indices are displayed.
Figure 5
   In order to analyze which cytokines are induced in PBMCs by the different milk samples (VTP1-VTP16), luminex-analyses were performed in the supernatants of PBMCs. Analysis of cytokine levels (y-axis: pg/ml) in supernatants of PBMC cultures from 6 non-allergic individuals (NA) and 5 cow's milk allergic patients (CMA) which had been stimulated with milk samples VTP1-VTP16 (1-16) or medium (17) are displayed.
Figure 6
   Tolerance experiments with a non-allergic individual (AB) were performed to show that peptides can tolerize T-cells in allergen-specific manner. Experiments in a non-allergic persons can also show tolerance because tolerance is measured as reduction of T cell reactivity (Ebner et al., J lmmunol 1995, Vol 154, pp 1932-1940). As long as a non-allergic person contains allergen-reactive T cells, tolerance to the allergen at the T cell level can be measured.
   PBMCs were isolated and T-cell- and antigen-presenting cell (APC)-enriched fractions were obtained by MACS-separation technology. T-cell fractions were preincubated over night with high amounts of peptides (αS1-casein peptide 1, αS1-casein peptide 2, αS1-casein peptide 3 and a control peptide, Phl p 5-peptide 1) or with an extensive whey hydrolysate (VTP8) or partial whey/casein hydrolysate (VTP16). On the next day the T-cells and the APCs were combined and stimulated with whole cow's milk protein samples (VTP13, VTP14) or with intact rαS1-casein.
   Two experiments were performed with the same individual. In panels a and c counts per minute (cpm; reflecting the proliferation of T cells) and in panels b and d stimulation indices (Sls) are displayed.
Figure 7
   Tolerance experiment with a non-allergic individual (KFI) evaluating milk hydrolsates for their ability to tolerize T cells. T-cell fractions were preincubated over night with high amounts of Nutramigen^{™} base (VTP11), extensive casein hydrlysate (VTP15), whole cow's milk whey protein (VTP14), Nutramigen^{™} (VTP3), synthetic peptides (αS1-casein peptide 4 and a control peptide, Phl p 5-Pep 1) or buffer (UCO). On the next day the T-cells and the APCs were combined and stimulated with milk samples (VTP14, VTP1). T cell stimulation is given either in counts per minute (cpm) or as stimulation index (SI). Cpms of individual experiments and the means thereof are displayed. Data obtained by stimulating with 10 µg VTP14 are highlighted.
Figure 8
   Profile of the casein derived peptides comprised in VTP3, VTP 11 and VTP 15 (Figure 1). The lonscore, the amino acid sequences of the peptides, the hydrophobicity score and the bitterness score are listed.

The Examples illustrate the invention.

### Example 1:

### Experimental procedures

### Biological materials and patients

Milk samples named VTP1-VTP16 were obtained from different suppliers and contained whole cow's milk protein, partially hydrolyzed, extensively hydrolyzed as well as amino acids formulations. cDNA coding for αS1-casein (rαS1-cas) was isolated by IgE immunoscreening from a cDNA expression library prepared from bovine mammary glands (Schulmeister et al., J Immunol. 2009). Recombinant allergens were expressed in *Escherichia coli* strain BL21 Codon Plus (DE3)-RIPL (Stratagene, La Jolla, CA) as hexahistidine-tagged proteins and purified by Ni²⁺ affinity chromatography (QIAGEN, Hilden, Germany). Recombinant Bet v1a was purchased from Biomay (Vienna, Austria).

Pasteurized cow's milk containing 3.5% fat was bought at a local market (NÖM, Austria, batch: 22 550 2:00) and natural cow's milk proteins were purchased from Sigma-Aldrich (Vienna, Austria).

Rabbit sera were obtained by immunizing rabbits three times with purified raS1-cas, raS2-cas, rβ-cas, rκ-cas, rα-1a, rβ-lg, and rlf (Charles, River, Kissleg, Germany).

Cow's milk allergic patients were selected according to a positive case history, positive skin-prick reactions or determination of specific IgE to cow's milk extract using the ImmunoCAP System (Phadia, Uppsala, Sweden).

Persons without any problems after milk consumption were recruited as controls. They comprised non-allergic as well as patients with IgE-mediated allergy to allergen sources other than milk.

### Characterization of the milk samples

The composition of the milk samples VTP1-VTP16 was assessed by SDS-PAGE and Coomassie Brilliant Blue staining (Biorad, Hercules, CA).

For immunoblot analysis 1µg aliquots of VTP1-VTP16 were dotted onto a nitrocellulose membrane (Schleicher & Schuell, Dassel, Germany). The nitrocellulose strips were blocked with PBST (PBS, 0.5% v/v Tween 20) and exposed to sera from milk allergic patients, healthy individuals or rabbit antisera diluted 1:10, 1:20 or 1:2000 in over night at 4°C. Bound human IgE antibodies were detected with ¹²⁵I-labelled anti-human IgE antibodies (IBL, Hamburg, Germany), diluted 1:15 or bound rabbit IgG with ¹²⁵I-labelled anti-rabbit IgG (Perkin Elmer, USA) diluted 1:2000 in PBST and visualized by autoradiography using Kodak XOMAT films with intensifying screens (Kodak, Austria) at -80°C.

Endotoxin levels of the milk samples used in this study were quantified by limulus amoebocyte lysate assay (Lonza, Basel, Switzerland) with a sensitivity range of 0.1 EU/ml -1.0 EU/ml according to the manufacturer's instructions.

### Rat Basophil Leukaemia (RBL) assays

For the quantification of IgE antibody-mediated, immediate-type reactions, huRBL cell mediator release assays were performed as described previously (Schulmeister et al., J Immunol. 2009, Vol 182(11), pp 7019-29). In brief, RBL cells (clone RBL-703/21) transfected with the human FcεRI receptor were incubated with sera from cow's milk allergic patients overnight. On the next day the cells were washed, 100 µl of milk components (concentration: 0.3 µg/ml) were added and incubated for 1 hour at 37°C, 7% CO₂, 95% humidity. Aliquots of the supernatants were mixed with assay solution (0.1 M citric acid or sodium citrate, pH 4.5 + 160µM 4-methyl umbelliferyl-N-acetyl-N-D-glucosamide) and incubated for 1 hour at 37°C, 7% CO₂ 95% humidity. Fluorescence was measured with a fluorescence microplate reader and specific release could be calculated. Values obtained with buffer alone were subtracted and the values exceeded 5% of total release were considered as positive.

### Cell preparation and lymphoproliferative assays

PBMCs from non-allergic individuals and cow's milk allergic patients were separated from heparinized blood by Ficoll density-gradient centrifugation (GE Healthcare, Uppsala, Sweden). PBMCs (2x10⁵ cells per well) were cultured in triplicates in 96-well plates (Nunclone; Nalgen Nunc International, Roskilde, Denmark) in 200 µl serum-free Ultra Culture medium (UltraCulture, Lonza, Verviers, Belgium) supplemented with 2mM L-glutamine (GIBCO, Auckland, NZ), 50 µM b-mercaptoethanol (GIBCO), and 0.1 mg/ml gentamicin (GIBCO). The cells were incubated at 37°C in a humidified atmosphere with 5% CO₂ for 7 days with or without different concentrations of various hydrolyzed samples. Cells were stimulated with different concentrations, 4U IL-2 per well (Roche, Mannheim, Germany) served as a positive control and medium alone served as a negative control. After 6 days of incubation, 0.5mCi 3H-thymidine (GE Healthcare) was added to each well for 16 h then the incorporated radioactivity was measured by liquid scintillation counting. Proliferation was expressed as counts per minute (c.p.m.; means of triplicates) using a microbeta scintillation counter (Wallac ADL, Freiburg, Germany). The stimulation index (SI) was calculated as quotient of c.p.m. with antigen and the medium control.

### Analysis of cytokine levels in supernatants

Cytokine levels (IL-2, IL-4, IL-5, IL-6, IL-10, IL-12, IL-13, IFN-γ, TNF-α, GM-CSF, TGF-β1, TGF-β2, and TGF-β3) were measured in supernatants collected from PBMC cultures at day 6 of culture using xMAP Luminex fluorescent bead-based technology (Luminex Corp., Austin, TX). The assays were performed according to the manufacturer's instructions (R&D Systems, Wiesbaden, Germany), and fluorescent signals were read on a Luminex 100 system (Luminex Corp.). The limits of detection were 3.2 pg/ml for IL-2, 3.5 pg/ml for IL-4, 1.9 pg/ml for IL-5, 5.5 pg/ml for IL-6, 3.1 pg/ml for IL-10, 57 pg/ml for IL-12, 47 pg/ml for IL-13, 2.9 pg/ml for IFN-γ, 5.3 pg/ml for TNF-α, 3.3 pg/ml for GM-CSF, 21 pg/ml for TGF-β1, 178 pg/ml for TGF-β2, and 5 pg/ml for TGF-β3.

### Tolerance induction experiments

For the tolerance induction experiment, PBMCs from healthy donors were isolated by Ficoll-density gradient, and the cells were further separated into two fractions. T-cells were enriched with a human Pan-T-cell isolation Kit (Miltenyi, Bergisch Gladbach, Germany), whereas antigen-presenting cells were enriched with human CD3 MicroBeads (Miltenyi, Bergisch Gladbach, Germany) by magnetic cell sorting. The purity of the cell fractions was confirmed by FACS analysis.

The cell fractions containing the T-cells were incubated overnight with different peptides or medium alone at 37°C with 5% CO₂ and 95% humidity, the antigen-presenting-cell fractions were kept overnight at the same conditions without any antigens. On the next day, the T-cell fractions were washed with DPBS (GIBCO) three times and the T-cell and antigen-presenting-cell fractions were combined. The cells (2x10⁵ cells per well) were cultured in triplicates in 96-well plates as described above. Cells were stimulated with different concentrations of milk samples, 4U IL-2 per well, or medium alone. The analysis was done as described for the lymphoproliferative assays.

Example 2:

### Results

Healthy non-allergic individuals exhibit a normal IgG but no IgE response to cow's milk allergens. In the experiments both quantitative (proliferation) and qualitative (i.e., cytokines) responses in cow's milk allergic individuals as well as healthy non-allergic individuals were compared.

The experiments identified small peptides in the hydrolysates which can block the T cell receptors and MHCII so that there is limited or no proliferation when there is a subsequent exposure to milk allergens. Thus, the experiments identified a fraction so that a tolerance to cow's milk allergens can be induced.

Another approach was to mix in the T cells that have been pre-exposed to peptides/hydrolysates in a culture of T-APC that have been preincubated and restimulated with whole protein preparations. This should demonstrate the capacity of peptides to inhibit/dampen the response to a whole milk protein challenge.

Up to now 6 non-allergic and 7 cow's milk allergic persons were tested regarding lymphoproliferative responses with the 16 cow's milk samples as well as with αS1casein-derived peptides and control antigens. Non-allergic individuals and cow's milk allergic patients induced comparable stimulation indices. The Nutramigen^{™} samples induced weaker T-cell proliferations compared to the other milk samples. Amino acid formulations induced the weakest responses. VTP13 and 14, identified by SDS-PAGE and mass spectrometry analysis as sources of intact cow's milk proteins, served as controls for tolerance experiments, because they induced strong lymphoproliferative responses. The culture supernatants from 11 of the tested individuals regarding the secretion of 13 cytokines were analyzed by luminex analysis.These results are extremely interesting and surprising for two reasons: First, the analysis of the cytokine levels showed that cytokines driving a Th2 differentiation (GM-CSF, IL-5 and IL-13) and pro-inflammatory cytokines (IL-6, TNF-alpha and IFN-gamma) are induced in low amounts by the Nutramigen^{™} samples (VTP3, 4, 11, 12, 15). By contrast, a strong induction of pro-inflammatory cytokines (e.g., IFN-gamma, TNF-alpha, IL-6) in the samples VTP2, VTP5 and VTP14 which are only partly hydrolysed or contain intact proteins has been found. In particular, the induction of IFN-gamma seems problematic because evidence exists that IFN-gamma can damage epithelial cells. Amounts of secreted cytokines were in the same range in experiments done with non-allergic and with allergic individuals, except of IFN-gamma, which was stronger upregulated in non-allergic individuals.

Certain cow's milk derived peptides (e.g. alpha S1casein) induce T cell proliferations in PBMC. Since any T cell-reactive peptide can also induce T cell tolerance under certain conditions (e.g., binding to the T cell receptor without appropriate co-stimulation, presence of large amounts of T cell-reactive peptides early in life-infancy) it is assumed that alphaS1casein-derived T cell-reactive peptides can be used for the induction of tolerance to identify additional T cell-reactive peptides. In this respect, tolerogenic activity of the alpha S1casein-derived peptides that will be generated synthetically was demonstrated. PBMC from reactive persons were splitted into T cell fraction and a T cell-depleted fraction (anti-CD3-coupled beads) containing the APCs. The T cell fraction were pre-incubated with the casein-derived peptides and control peptides (grass pollen allergen Bet v1, Phl p 5-derived peptides), washed and then exposed to the APC fraction with the peptides. Using this assay it was possible to demonstrate the milk-specific tolerogenic activity of these casein-derived peptides.

In an effort to search for tolerogenic peptides the culture conditions were successfully established. In a next step one αS1 casein-derived candidate peptide which showed in two independent experiments a suppression of T cell responses was identified. The alphaS1-casein-derived peptide 3, when added to an isolated T-cell fraction suppressed the proliferation in the combined T-cell-APC fraction when re-stimulated with whey protein concentrate VTP14 or rαS1-casein. A similar result was obtained for peptide 4. Further, the potential suppressive effects of casein hydrolysates VTP3 (Nutramigen^{™}), VTP11, VTP14 and VTP15 on whole cow's milk protein induced proliferation were studied and it was found that in particular preincubation with Nutramigen^{™} (VTP3) was able to induce tolerance.

## Claims

1. A nutritional formulation or supplement comprising an extensively hydrolyzed cow's milk peptide-containing hydrolysate and/or a peptide-containing fraction of the extensively hydrolyzed cow's milk peptide-containing hydrolysate and/or one or more T cell epitope-containing peptides isolated from the extensively hydrolyzed cow's milk peptide-containing hydrolysate for use in the induction of tolerance to cow's milk, a protein contained in cow's milk or an allergen contained in cow's milk in a human subject, wherein said peptides contained in the hydrolysate or fraction of hydrolysate comprise T cell epitope-containing peptides or wherein said one or more peptides are T cell epitope-containing peptides, wherein said T cell epitope-containing peptides are capable of driving the immune reaction upon intake of the nutritional formulation towards tolerance and wherein the extensively hydrolyzed cow's milk peptide-containing hydrolysate comprises less than 1% of peptides having a size of greater than 1.5kD.

2. The nutritional formulation or supplement of claim 1, wherein the one or more peptides have the amino acid sequence of SEQ ID NO: 3 and/or 4.

3. The nutritional formulation or supplement of claim 1, wherein the extensively hydrolyzed cow's milk peptide-containing hydrolysate is an extensively hydrolyzed bovine casein-containing hydrolysate.

4. The nutritional formulation or supplement of any of claims 1 to 3, wherein the human subject is a child or juvenile.

5. The nutritional formulation or supplement of claims 1 to 3, wherein the human subject is an adult.

6. The nutritional formulation or supplement of any of claims 1 to 5, wherein the human subject has a cow's milk allergy.

7. The nutritional formulation or supplement of any of claims 1 to 6, wherein the nutritional formulation additionally comprises one or more of carbohydrates, nucleic acids, lipids, minerals, anabolic nutrients, vitamins, antioxidants, probiotic bacterial strains and lipotropic agents.

8. The nutritional formulation or supplement of any of claims 1 to 7, wherein the tolerance is induced transiently.

9. The nutritional formulation or supplement of any of claims 1 to 8, wherein the peptides consist of about 5 to 50 amino acids.

10. The nutritional formulation or supplement of any of claims 1 to 9, wherein the T cell epitope-containing peptides are capable of down regulating inflammatory cytokines upon intake of the nutritional formulation.

## Patentansprüche

1. Nahrungs-Zusammensetzung oder -Ergänzung, umfassend ein ausgiebig hydrolysiertes Kuhmilch-Peptid-enthaltendes Hydrolysat und/oder eine Peptid-enthaltende Fraktion des ausgiebig hydrolysierten Kuhmilch-Peptid-enthaltenden Hydrolysats und/oder ein oder mehrere T-Zell-Epitop-enthaltende Peptide, die aus dem ausgiebig hydrolysierten Kuhmilch-Peptid-enthaltenden Hydrolysat isoliert wurden, zur Verwendung bei der Induktion von Toleranz gegenüber Kuhmilch, einem Protein, das in Kuhmilch enthalten ist, oder einem Allergen, das in Kuhmilch enthalten ist, in einem menschlichen Individuum, wobei die in dem Hydrolysat oder der Fraktion enthaltenen Peptide T-Zell-Epitop-enthaltende Peptide umfassen oder wobei das eine oder die mehreren Peptide T-Zell-Epitop-enthaltende Peptide sind, wobei die T-Zell-Epitop-enthaltenden Peptide eine auf Toleranz gerichtete Immunantwort nach Einnahme der Nahrungs-Zusammensetzung oder -Ergänzung herbeiführen können und wobei das ausgiebig hydrolysierte Kuhmilch-Peptid-enthaltende Hydrolysat weniger als 1 % Peptide umfasst, die eine Größe von mehr als 1,5kD haben.

2. Nahrungs-Zusammensetzung oder -Ergänzung nach Anspruch 1, wobei das eine oder die mehreren Peptide die Aminosäuresequenz der SEQ ID NO:3 und/oder 4 haben.

3. Nahrungs-Zusammensetzung oder -Ergänzung nach Anspruch 1, wobei das ausgiebig hydrolysierte Kuhmilch-Peptid-enthaltende Hydrolysat ein ausgiebig hydrolysiertes Rinder-Casein-enthaltendes Hydrolysat ist.

4. Nahrungs-Zusammensetzung oder -Ergänzung nach einem der Ansprüche 1 bis 3, wobei das menschliche Individuum ein Kind oder ein Jugendlicher ist.

5. Nahrungs-Zusammensetzung oder -Ergänzung nach einem der Ansprüche 1 bis 3, wobei das menschliche Individuum ein Erwachsener ist.

6. Nahrungs-Zusammensetzung oder -Ergänzung nach einem der Ansprüche 1 bis 5, wobei das menschliche Individuum eine Kuhmilchallergie hat.

7. Nahrungs-Zusammensetzung oder -Ergänzung nach einem der Ansprüche 1 bis 6, wobei die Nahrungsmittel-Zusammensetzung weiterhin einen oder mehrere Bestandteil(e) ausgewählt aus Kohlenhydraten, Nukleinsäuren, Lipiden, Mineralien, anabolischen Nährstoffen, Vitaminen, Antioxidantien, probiotischen Bakterienstämmen und lipotropen Wirkstoffen umfasst.

8. Nahrungs-Zusammensetzung oder -Ergänzung nach einem der Ansprüche 1 bis 7, wobei die Toleranz transient induziert wird.

9. Nahrungs-Zusammensetzung oder -Ergänzung nach einem der Ansprüche 1 bis 8, wobei die Peptide aus etwa 5 bis 50 Aminosäuren bestehen.

10. Nahrungs-Zusammensetzung oder -Ergänzung nach einem der Ansprüche 1 bis 9, wobei die T-Zell-Epitop-enthaltenden Peptide inflammatorische Zytokine nach der Einnahme der Nahrungs-Zusammensetzung herunterregulieren können.

## Revendications

1. Formulation ou complément nutritionnel comprenant un hydrolysat contenant des peptides de lait de vache intensivement hydrolysé et/ou une fraction contenant des peptides de l'hydrolysat contenant des peptides de lait de vache intensivement hydrolysé et/ou un ou plusieurs peptides contenant des épitopes de lymphocytes T isolés à partir de l'hydrolysat contenant des peptides de lait de vache intensivement hydrolysé pour une utilisation dans l'induction d'une tolérance au lait de vache, à une protéine contenue dans le lait de vache ou à un allergène contenu dans le lait de vache chez un sujet humain, dans lequel lesdits peptides contenus dans l'hydrolysat ou la fraction de l'hydrolysat comprennent des peptides contenant des épitopes de lymphocytes T ou dans lequel lesdits un ou plusieurs peptides sont des peptides contenant des épitopes de lymphocytes T, où lesdits peptides contenant des épitopes de lymphocytes T sont capables de diriger la réponse immunitaire lors de la prise de la formulation nutritionnelle vers une tolérance et où l'hydrolysat contenant des peptides de lait de vache intensivement hydrolysé comprend moins de 1 % de peptides ayant une taille supérieure à 1,5 kD.

2. Formulation ou complément nutritionnelle selon la revendication 1, dans laquelle les un ou plusieurs peptides ont la séquence d'acides aminés de SEQ ID NO : 3 et/ou 4.

3. Formulation ou complément nutritionnelle selon la revendication 1, dans laquelle l'hydrolysat contenant des peptides de lait de vache intensivement hydrolysé est un hydrolysat contenant de la caséine bovine intensivement hydrolysée.

4. Formulation ou complément nutritionnelle selon l'une quelconque des revendications 1 à 3, où le sujet humain est un enfant ou un adolescent.

5. Formulation ou complément nutritionnelle selon les revendications 1 à 3, où le sujet humain est un adulte.

6. Formulation ou complément nutritionnelle selon l'une quelconque des revendications 1 à 5, où le sujet humain présente une allergie au lait de vache.

7. Formulation ou complément nutritionnelle selon l'une quelconque des revendications 1 à 6, où la formulation nutritionnelle comprend en outre un ou plusieurs des glucides, acides nucléiques, lipides, minéraux, nutriments anaboliques, vitamines, antioxydants, souches bactériennes probiotiques et agents lipotropes.

8. Formulation ou complément nutritionnelle selon l'une quelconque des revendications 1 à 7, où la tolérance est induite de manière transitoire.

9. Formulation ou complément nutritionnelle selon l'une quelconque des revendications 1 à 8, dans laquelle les peptides sont constitués d'environ 5 à 50 acides aminés.

10. Formulation ou complément nutritionnelle selon l'une quelconque des revendications 1 à 9, dans laquelle les peptides contenant des épitopes de lymphocytes T sont capables de réguler à la baisse les cytokines inflammatoires lors de la prise de la formulation nutritionnelle.


### SEQUENCE LISTING

<110> Mead Johnson & Company
<120> NUTRITIONAL FORMULATION COMPRISING A COW'S MILK PEPTIDE-CONTAINING
   HYDROLYSATE AND/OR PEPTIDES DERIVED THEREOF FOR TOLERANCE INDUCTION
<130> R3074 EP
<160> 12
<170> PatentIn version 3.3
<210> 1
   <211> 32
   <212> PRT
   <213> BOS taurus
<400> 1
   Arg Pro Lys His Pro Ile Lys His Gln Gly Leu Pro Gln Glu Val Leu
   1               5                   10                  15
   Asn Glu Asn Leu Leu Arg Phe Phe Val Ala Pro Phe Pro Glu Val Cys
               20                  25                  30
<210> 2
   <211> 33
   <212> PRT
   <213> BOS taurus
<400> 2
   Phe Gly Lys Glu Lys Val Asn Glu Leu Ser Lys Asp Ile Gly Ser Glu
   1 5 10 15
   Ser Thr Glu Asp Gln Ala Met Glu Asp Ile Lys Gln Met Glu Ala Glu
   20 25 30
   Ser
<210> 3
   <211> 36
   <212> PRT
   <213> BOS taurus
<400> 3
   Ile Ser Ser Ser Glu Glu Ile Val Pro Asn Ser Val Glu Gln Lys His
   1 5 10 15
   Ile Gln Lys Glu Asp Val Pro Ser Glu Arg Tyr Leu Gly Tyr Glu Gln
   20 25 30
   Leu Leu Arg Cys
   35
<210> 4
   <211> 34
   <212> PRT
   <213> BOS taurus
<400> 4
   Cys Leu Lys Lys Tyr Lys Val Pro Gln Leu Glu Ile Val Pro Asn Ser
   1 5 10 15
   Ala Glu Glu Arg Leu His Ser Met Lys Glu Gly Ile His Ala Gln Gln
   20 25 30
   Lys Glu
<210> 5
   <211> 35
   <212> PRT
   <213> BOS taurus
<400> 5
   Cys Pro Met Ile Gly Val Asn Gln Glu Leu Ala Tyr Phe Tyr Pro Glu
   1 5 10 15
   Leu Phe Arg Gln Phe Tyr Gln Leu Asp Ala Tyr Pro Ser Gly Ala Trp
   20 25 30
   Tyr Tyr Val
   35
<210> 6
   <211> 33
   <212> PRT
   <213> BOS taurus
<400> 6
   Pro Leu Gly Thr Gln Tyr Thr Asp Ala Pro Ser Phe Ser Asp Ile Pro
   1 5 10 15
   Asn Pro Ile Gly Ser Glu Asn Ser Glu Lys Thr Thr Met Pro Leu Trp
   20 25 30
   Cys
<210> 7
   <211> 205
   <212> PRT
   <213> BOS taurus
<400> 7
   Arg Pro Lys His Pro Ile Lys His Gln Gly Leu Pro Gln Glu Val Leu
   1 5 10 15
   Asn Glu Asn Leu Leu Arg Phe Phe Val Ala Pro Phe Pro Glu Val Phe
   20 25 30
   Gly Lys Glu Lys Val Asn Glu Leu Ser Lys Asp Ile Gly Ser Glu Ser
           35                  40                  45
   Thr Glu Asp Gln Ala Met Glu Asp Ile Lys Gln Met Glu Ala Glu Ser
       50                  55                  60
   Ile Ser Ser Ser Glu Glu Ile Val Pro Asn Ser Val Glu Gln Lys His
   65                  70                  75                  80
   Ile Gln Lys Glu Asp Val Pro Ser Glu Arg Tyr Leu Gly Tyr Leu Glu
                   85                  90                  95
   Gln Leu Leu Arg Leu Lys Lys Tyr Lys Val Pro Gln Leu Glu Ile Val
               100                 105                 110
   Pro Asn Ser Ala Glu Glu Arg Leu His Ser Met Lys Glu Gly Ile His
           115                 120                 125
   Ala Gln Gln Lys Glu Pro Met Ile Gly Val Asn Gln Glu Leu Ala Tyr
       130                 135                 140
   Phe Tyr Pro Glu Leu Phe Arg Gln Phe Tyr Gln Leu Asp Ala Tyr Pro
   145                 150                 155                 160
   Ser Gly Ala Trp Tyr Tyr Val Pro Leu Gly Thr Gln Tyr Thr Asp Ala
                   165                 170                 175
   Pro Ser Phe Ser Asp Ile Pro Asn Pro Ile Gly Ser Glu Asn Ser Glu
               180                 185                 190
   Lys Thr Thr Met Pro Leu Trp His His His His His His
           195                 200                 205
<210> 8
   <211> 213
   <212> PRT
   <213> BOS taurus
<400> 8
   Lys Asn Thr Met Glu His Val Ser Ser Ser Glu Glu Ser Ile Ile Ser
   1               5                   10                  15
   Gln Glu Thr Tyr Lys Gln Glu Lys Asn Met Ala Ile Asn Pro Ser Lys
               20                  25                  30
   Glu Asn Leu Cys Ser Thr Phe Cys Lys Glu Val Val Arg Asn Ala Asn
           35                  40                  45
   Glu Glu Glu Tyr Ser Ile Gly Ser Ser Ser Glu Glu Ser Ala Glu Val
       50                  55                  60
   Ala Thr Glu Glu Val Lys Ile Thr Val Asp Asp Lys His Tyr Gln Lys
   65 70 75 80
   Ala Leu Asn Glu Ile Asn Gln Phe Tyr Gln Lys Phe Pro Gln Tyr Leu
   85 90 95
   Gln Tyr Leu Tyr Gln Gly Pro Ile Val Leu Asn Pro Trp Asp Gln Val
   100 105 110
   Lys Arg Asn Ala Val Pro Ile Thr Pro Thr Leu Asn Arg Glu Gln Leu
   115 120 125
   Ser Thr Ser Glu Glu Asn Ser Lys Lys Thr Val Asp Met Glu Ser Thr
   130 135 140
   Glu Val Phe Thr Lys Lys Thr Lys Leu Thr Glu Glu Glu Lys Asn Arg
   145 150 155 160
   Leu Asn Phe Leu Lys Lys Ile Ser Gln Arg Tyr Gln Lys Phe Ala Leu
   165 170 175
   Pro Gln Tyr Leu Lys Thr Val Tyr Gln His Gln Lys Ala Met Lys Pro
   180 185 190
   Trp Ile Gln Pro Lys Thr Lys Val Ile Pro Tyr Val Arg Tyr Leu His
   195 200 205
   His His His His His
   210
<210> 9
   <211> 159
   <212> PRT
   <213> Betula verrucosa
<400> 9
   Gly Val Phe Asn Tyr Glu Thr Glu Thr Thr Ser Val Ile Pro Ala Ala
   1 5 10 15
   Arg Leu Phe Lys Ala Phe Ile Leu Asp Gly Asp Asn Leu Phe Pro Lys
   20 25 30
   Val Ala Pro Gln Ala Ile Ser Ser Val Glu Asn Ile Glu Gly Asn Gly
   35 40 45
   Gly Pro Gly Thr Ile Lys Lys Ile Ser Phe Pro Glu Gly Phe Pro Phe
   50 55 60
   Lys Tyr Val Lys Asp Arg Val Asp Glu Val Asp His Thr Asn Phe Lys
   65 70 75 80
   Tyr Asn Tyr Ser Val Ile Glu Gly Gly Pro Ile Gly Asp Thr Leu Glu
   85 90 95
   Lys Ile Ser Asn Glu Ile Lys Ile Val Ala Thr Pro Asp Gly Gly Ser
   100 105 110
   Ile Leu Lys Ile Ser Asn Lys Tyr His Thr Lys Gly Asp His Glu Val
   115 120 125
   Lys Ala Glu Gln Val Lys Ala Ser Lys Glu Met Gly Glu Thr Leu Leu
   130 135 140
   Arg Ala Val Glu Ser Tyr Leu Leu Ala His Ser Asp Ala Tyr Asn
   145 150 155
<210> 10
   <211> 32
   <212> PRT
   <213> Phleum pratense
<400> 10
   Cys Gly Ala Ala Ser Asn Lys Ala Phe Ala Glu Gly Leu Ser Gly Glu
   1 5 10 15
   Pro Lys Gly Ala Ala Glu Ser Ser Ser Lys Ala Ala Leu Thr Ser Lys
   20 25 30
<210> 11
   <211> 199
   <212> PRT
   <213> BOS taurus
<400> 11
   Arg Pro Lys His Pro Ile Lys His Gln Gly Leu Pro Gln Glu Val Leu
   1               5                   10                  15
   Asn Glu Asn Leu Leu Arg Phe Phe Val Ala Pro Phe Pro Glu Val Phe
               20                  25                  30
   Gly Lys Glu Lys Val Asn Glu Leu Ser Lys Asp Ile Gly Ser Glu Ser
           35                  40                  45
   Thr Glu Asp Gln Ala Met Glu Asp Ile Lys Gln Met Glu Ala Glu Ser
       50                  55                  60
   Ile Ser Ser Ser Glu Glu Ile Val Pro Asn Ser Val Glu Gln Lys His
   65                  70                  75                  80
   Ile Gln Lys Glu Asp Val Pro Ser Glu Arg Tyr Leu Gly Tyr Leu Glu
                   85                  90                  95
   Gln Leu Leu Arg Leu Lys Lys Tyr Lys Val Pro Gln Leu Glu Ile Val
               100                 105                 110
   Pro Asn Ser Ala Glu Glu Arg Leu His Ser Met Lys Glu Gly Ile His
           115                 120                 125
   Ala Gln Gln Lys Glu Pro Met Ile Gly Val Asn Gln Glu Leu Ala Tyr
       130                 135                 140
   Phe Tyr Pro Glu Leu Phe Arg Gln Phe Tyr Gln Leu Asp Ala Tyr Pro
   145                 150                 155                 160
   Ser Gly Ala Trp Tyr Tyr Val Pro Leu Gly Thr Gln Tyr Thr Asp Ala
                   165                 170                 175
   Pro Ser Phe Ser Asp Ile Pro Asn Pro Ile Gly Ser Glu Asn Ser Glu
               180                 185                 190
   Lys Thr Thr Met Pro Leu Trp
           195
<210> 12
   <211> 207
   <212> PRT
   <213> BOS taurus
<400> 12
   Lys Asn Thr Met Glu His Val Ser Ser Ser Glu Glu Ser Ile Ile Ser
   1               5                   10                  15
   Gln Glu Thr Tyr Lys Gln Glu Lys Asn Met Ala Ile Asn Pro Ser Lys
               20                  25                  30
   Glu Asn Leu Cys Ser Thr Phe Cys Lys Glu Val Val Arg Asn Ala Asn
           35                  40                  45
   Glu Glu Glu Tyr Ser Ile Gly Ser Ser Ser Glu Glu Ser Ala Glu Val
       50                  55                  60
   Ala Thr Glu Glu Val Lys Ile Thr Val Asp Asp Lys His Tyr Gln Lys
   65                  70                  75                  80
   Ala Leu Asn Glu Ile Asn Gln Phe Tyr Gln Lys Phe Pro Gln Tyr Leu
                   85                  90                  95
   Gln Tyr Leu Tyr Gln Gly Pro Ile Val Leu Asn Pro Trp Asp Gln Val
               100                 105                 110
   Lys Arg Asn Ala Val Pro Ile Thr Pro Thr Leu Asn Arg Glu Gln Leu
           115                 120                 125
   Ser Thr Ser Glu Glu Asn Ser Lys Lys Thr Val Asp Met Glu Ser Thr
       130                 135                 140
   Glu Val Phe Thr Lys Lys Thr Lys Leu Thr Glu Glu Glu Lys Asn Arg
   145                 150                 155                 160
   Leu Asn Phe Leu Lys Lys Ile Ser Gln Arg Tyr Gln Lys Phe Ala Leu
                   165                 170                 175
   Pro Gln Tyr Leu Lys Thr Val Tyr Gln His Gln Lys Ala Met Lys Pro
               180                 185                 190
   Trp Ile Gln Pro Lys Thr Lys Val Ile Pro Tyr Val Arg Tyr Leu
           195                 200                 205